# EUROPEAN PATENT APPLICATION

(11) **EP 4 635 549 A1**
(43) Date of publication of application: **22.10.2025**
(21) Application number: 25170447.4
(22) Date of filing: 14.04.2025
(51) Int. Cl.: A61M 25/01, B23K 37/04

(54) **PULL RING HOLDER FOR A PULL WIRE ASSEMBLY MACHINE**

(30) Priority: 16.04.2024 US 202463634516 P; 16.04.2024 US 202463634510 P; 16.04.2024 US 202463634509 P; 16.04.2024 US 202463634514 P; 19.06.2024 US 202418747655
(71) Applicant: TE Connectivity Solutions GmbH, 8200 Schaffhausen (CH); Tyco Electronics Mexico, S. De R.L. De C.V., 54060 Tlalnepantla (MX); Tyco Electronics (Shanghai) Co., Ltd., Pilot Free Trade Zone Shanghai 200131 (CN)
(72) Inventor: REYES, Abraham, 83118 HERMOSILLO (MX); CLAYTON, Jorge Enrique, 83118 HERMOSILLO (MX); LU, Roberto Francisco-Yi, Middletown, 17057 (US); JAUREGUI BUENROSTRO, Manuel, 83118 HERMOSILLO (MX); CHÁVEZ GUILLÉN, Ana Michelle, 83118 HERMOSILLO (MX); HU, Lvhai, SHANGHAI, 200131 (CN)
(74) Representative: Ashton, Gareth Mark

(57) **Abstract**

A ring holder (306) for a pull wire assembly machine (100) configured to assemble a pull wire (20) to a pull ring (30) is provided. The ring holder includes a ring mandrel (380) having a first mandrel member (390) and a second mandrel (392) member meeting at a seam (391). The ring mandrel includes a ring mount (382) at a front of the ring mandrel configured to hold the pull ring. The first and second mandrel members are movable relative to each other to expand a size of the ring mount to hold the pull ring on the ring mount and the first and second mandrel members being movable relative to each other to reduce a size of the ring mount to release the pull ring from the ring mount.

## Description

This application claims the benefit of U.S. Provisional Application No. 63/634,509, filed 16-April-2024, U.S. Provisional Application No. 63/634,510, filed 16-April-2024, U.S. Provisional Application No. 63/634,514, filed 16-April-2024, U.S. Provisional Application No. 63/634,516, filed 16-April-2024, and U.S. Non-Provisional Application No. 18/747,655 filed 19 June 2024, the subject matter of which are herein incorporated by reference in their entirety.

The subject matter herein relates generally to an assembly machine for a pull wire assembly.

Pull wire assemblies are used in various applications. The pull wire assembly includes a pull ring and one or more pull wires attached to the pull ring. The pull wires are very small, such as having a diameter of .0078 inches (0.198mm) or .012 inches (0.305mm), which are designed to fit in a slot in the pull ring, which are also very small, such as having a diameter of 0.06 inches (1.524mm) or 0.178 inches (4.52 mm). The pull wires assemblies are manually assembled relying on the operator carefully handling the components to load the pull ring and the pull wire into a fixture using miniature pliers. The operator observes the assembly process through a microscope. The components need to be precisely positioned to achieve a reliable connection through a laser welding process. The assembly process is time consuming, typically taking approximately 50 seconds for a single wire assembly or 75 seconds for a dual wire assembly. Additionally, the manual assembly process places significant physical strain on the operator, leading to fatigue and an increased risk of producing defective parts. Additionally, after assembly, the operator again must carefully remove the pull wire assembly from the fixturing to avoid pulling or bending the wires near the joint and along the entire length of the assembly.

A need remains for an automated machine for assembling and processing pull wire assemblies.

In one embodiment, a ring holder for a pull wire assembly machine configured to assemble a pull wire to a pull ring is provided. The ring holder includes a ring mandrel having a first mandrel member and a second mandrel member meeting at a seam. The ring mandrel includes a ring mount at a front of the ring mandrel configured to hold the pull ring. The first and second mandrel members are movable relative to each other to expand a size of the ring mount to hold the pull ring on the ring mount and the first and second mandrel members being movable relative to each other to reduce a size of the ring mount to release the pull ring from the ring mount.

The invention will be described by way of example with reference to the accompanying Figures of which:
Figure 1 is a perspective view of a pull wire assembly machine in accordance with an exemplary embodiment.
Figure 2 is a front view of the pull wire assembly machine in accordance with an exemplary embodiment.
Figure 3 is a left side view of the pull wire assembly machine in accordance with an exemplary embodiment.
Figure 4 is a right side view of the pull wire assembly machine in accordance with an exemplary embodiment the pull wire assembly machine is used to assemble a pull wire assembly 10 from a pull wire 20 and a pull ring 30.
Figure 5 is a perspective view of the pull wire assembly in accordance with an exemplary embodiment.
Figure 6 is a top view of the pull wire assembly in accordance with an exemplary embodiment.
Figure 7 is an enlarged view of a portion of the pull wire assembly in accordance with an exemplary embodiment showing the pull wire laser welded to the pull ring.
Figure 8 is a top view of the pull wire assembly in accordance with an exemplary embodiment.
Figure 9 is a side view of the pull wire assembly in accordance with an exemplary embodiment.
Figure 10 is an enlarged view of a portion of the pull wire assembly in accordance with an exemplary embodiment showing the pull wire laser welded to the pull ring.
Figure 11 illustrates an assembly method for assembling the pull wire assembly in accordance with an exemplary embodiment.
Figure 12 is a perspective view of the pull wire assembly machine in accordance with an exemplary embodiment.
Figure 13 is a front perspective view of the wire placement system in accordance with an exemplary embodiment.
Figure 14 is a rear perspective view of the wire placement system in accordance with an exemplary embodiment.
Figure 15 is an enlarged view of a portion of the wire placement system in accordance with an exemplary embodiment.
Figure 16 is an enlarged view of a portion of the wire placement system in accordance with an exemplary embodiment.
Figure 17 shows a method of loading the pull wire into the assembly zone of the pull wire assembly machine accordance with an exemplary embodiment.
Figure 18 is a perspective view of internal components of the systems of the pull wire assembly machine in accordance with an exemplary embodiment.
Figure 19 is a perspective view of the ring feeder in accordance with an exemplary embodiment.
Figure 20 is a perspective view of the ring manipulator in accordance with an exemplary embodiment.
Figure 21 is a perspective view of the end effector of the ring manipulator in accordance with an exemplary embodiment.
Figure 22 is a perspective view of the large diameter vacuum nozzle in accordance with an exemplary embodiment.
Figure 23 is an end view of the large diameter vacuum nozzle in accordance with an exemplary embodiment.
Figure 24 is a perspective view of the small diameter vacuum nozzle in accordance with an exemplary embodiment.
Figure 25 is an end view of the small diameter vacuum nozzle in accordance with an exemplary embodiment.
Figure 26 is a front perspective view of the ring holder in accordance with an exemplary embodiment.
Figure 27 is a rear perspective view of the ring holder in accordance with an exemplary embodiment.
Figure 28 is a front perspective view of a portion of the ring holder showing the ring mandrel in accordance with an exemplary embodiment.
Figure 29 is a side view of the ring mandrel in accordance with an exemplary embodiment.
Figure 30 is a front perspective view of a portion of the ring mandrel in accordance with an exemplary embodiment.
Figure 31 is a front perspective view of the laser weld system in accordance with an exemplary embodiment.
Figure 32 is a rear perspective view of the laser weld system in accordance with an exemplary embodiment.
Figure 33 illustrates an assembly method for assembling the pull wire assembly in accordance with an exemplary embodiment showing vision tasks performed by the vision system.
Figure 34 is a front perspective view of the pull wire assembly removal system in accordance with an exemplary embodiment.
Figure 35 is a perspective view of a portion of the pull wire assembly removal system showing the transport device and the sorting tray assembly located adjacent the transport device accordance with an exemplary embodiment.
Figure 36 is an enlarged view of a portion of the transport device showing the holding member in accordance with an exemplary embodiment.
Figure 37 is a perspective view of the holding member in accordance with an exemplary embodiment.
Figure 38 is a top view of the holding member in accordance with an exemplary embodiment.
Figure 39 is a perspective view of the storage tray assembly in accordance with an exemplary embodiment.
Figure 40 illustrates a removal method for removing the pull wire assemblies from the pull wire assembly machine accordance with an exemplary embodiment.

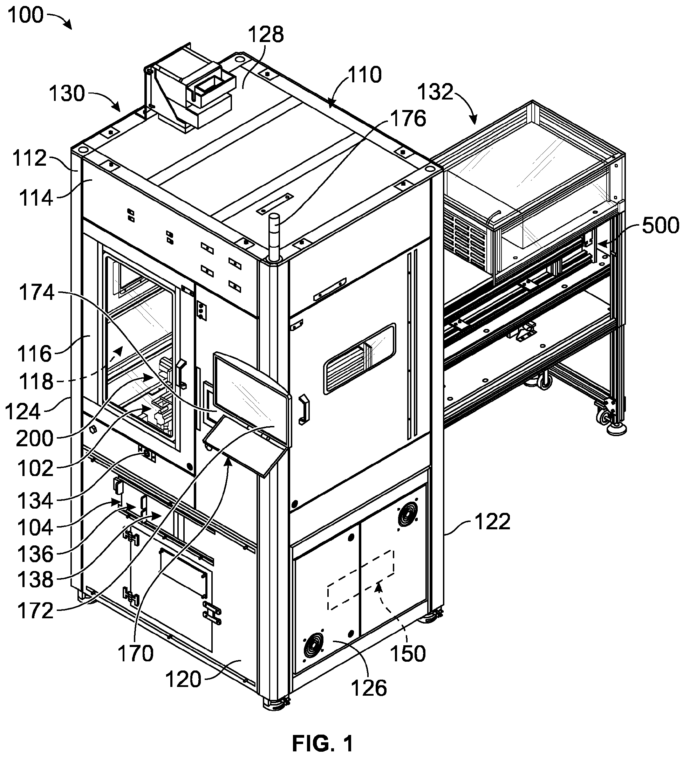

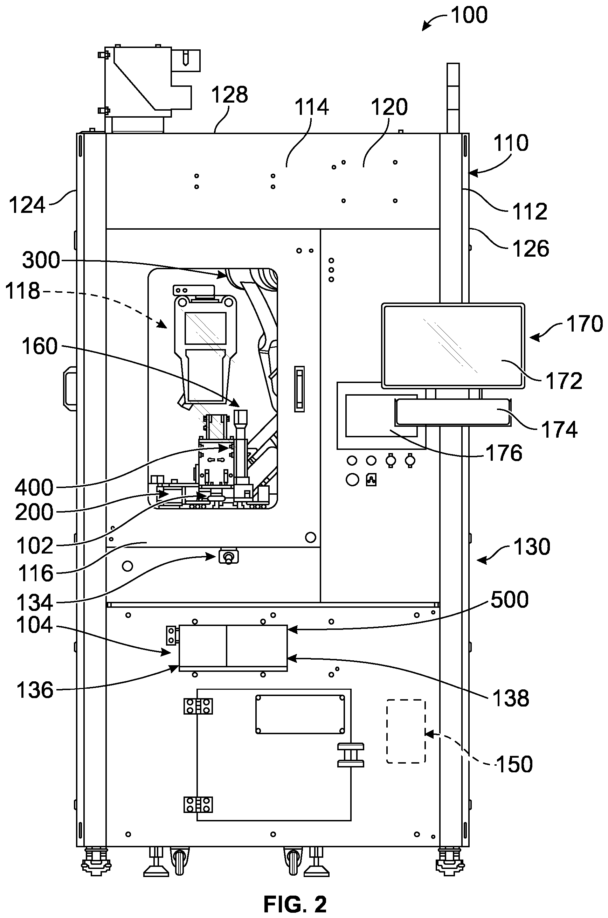

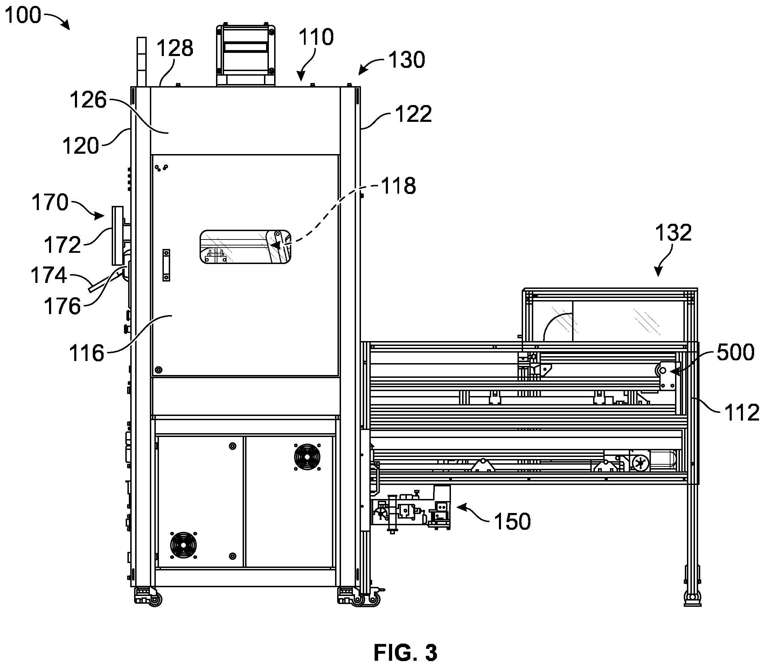

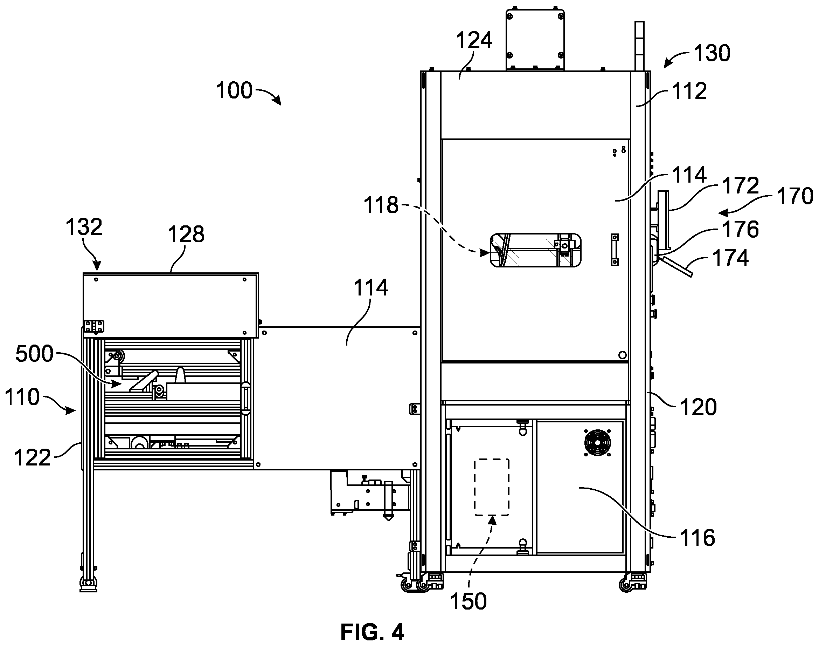

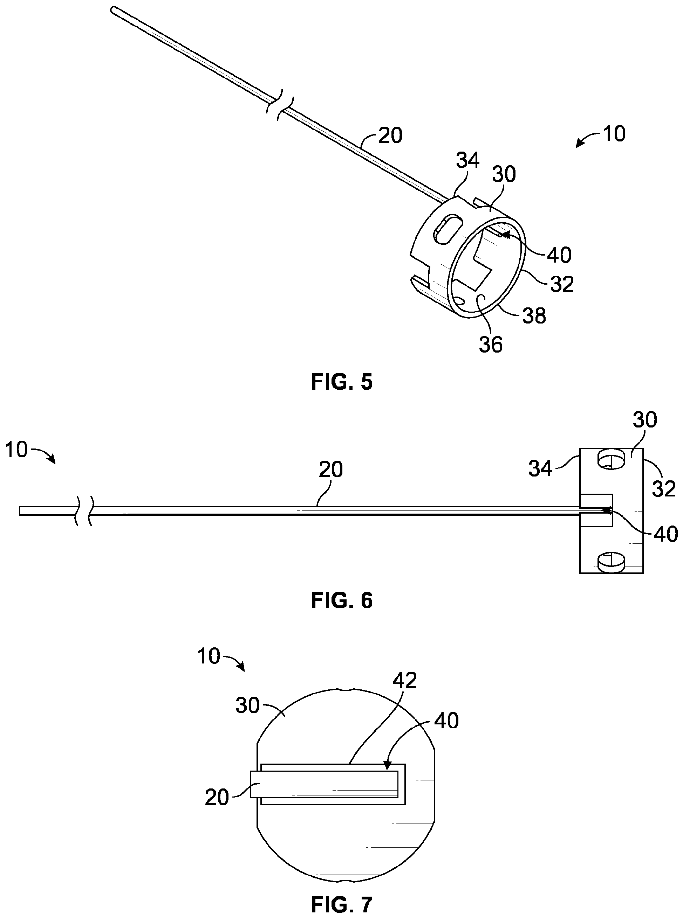

Figure 1 is a perspective view of a pull wire assembly machine 100 in accordance with an exemplary embodiment. Figure 2 is a front view of the pull wire assembly machine 100 in accordance with an exemplary embodiment. Figure 3 is a left side view of the pull wire assembly machine 100 in accordance with an exemplary embodiment. Figure 4 is a right side view of the pull wire assembly machine 100 in accordance with an exemplary embodiment the pull wire assembly machine 100 is used to assemble a pull wire assembly 10 from a pull wire 20, such as a flexible wire, and a pull ring 30 (examples shown in Figures 5-10). The pull wire assembly machine 100 is used to automate the assembly process to assemble the pull wire 20 to the pull ring 30. For example, the pull wire assembly machine 100 may position the pull wire 20 and the pull ring 30 for laser welding the pull wire 20 to the pull ring 30.

In an exemplary embodiment, the pull wire assembly machine 100 includes a wire placement system 200 for placing the pull wire 20 at an assembly zone 102 of the pull wire assembly machine 100, a ring placement system 300 for placing the pull ring 30 at the assembly zone 102, a laser weld system 400 at the assembly zone 102 for laser welding the pull wire 20 to the pull ring 30 to form the pull wire assembly 10, and a pull wire assembly removal system 500 at a removal zone 104 of the pull wire assembly machine 100 for removing the formed pull wire assembly 10 from the pull wire assembly machine 100.

In an exemplary embodiment, the pull wire assembly machine 100 includes a cabinet 110 that holds the components of the pull wire assembly machine 100, such as the various components of the wire placement system 200, the ring placement system 300, the laser weld system 400, and the pull wire assembly removal system 500. The cabinet 110 surrounds the components to protect the components. The cabinet 110 surrounds the components to prevent injury to the operator during operation of the pull wire assembly machine 100.

In an exemplary embodiment, the cabinet 110 includes a frame 112 and panels 114 coupled to the frame 112. The cabinet 110 includes access doors 116 that provide access to interior compartments 118 of the cabinet 110. The cabinet 110 includes a front 120 and a rear 122. The cabinet 110 includes a first side 124 and a second side 126 opposite the first side 124. The cabinet 110 includes a top 128. The bottom of the cabinet 110 may rest on a floor surface. In an exemplary embodiment, the cabinet 110 includes the panels 114 at the front 120, the rear 122, the right side 124, the left side 126, and the top 128. The doors 116 may be provided at the front 120 and/or the rear 122 and/or the right side 124 and/or the left side 126 and/or the top 128.

In an exemplary embodiment, the cabinet 110 includes a front portion 130 and a rear portion 132. The front portion 130 may house various components, such as components of the wire placement system 200 and/or the ring placement system 300 and/or the laser weld system 400 and/or the removal system 500. The rear portion 132 may house various components, such as components of the wire placement system 200 and/or the ring placement system 300 and/or the laser weld system 400 and/or the removal system 500.

In an exemplary embodiment, the pull wire assembly machine 100 includes a wire load port 134. The pull wire 20 is configured to be loaded into the pull wire assembly machine 100 through the wire load port 134. In the illustrated embodiment, the wire load port 134 is located at the front 120. Other locations are possible in alternative embodiments. In an exemplary embodiment, the pull wire 20 may be manually loaded into the pull wire assembly machine 100 through the wire load port 134. In alternative embodiments, the pull wire assembly machine 100 may include an automated system for automatically loading the pull wire 20 into the assembly zone 102. For example, a stock of the pull wires 20 may be held in the pull wire assembly machine 100 and a wire feeder may feed the pull wire 20 into the assembly zone 102.

**In** an exemplary embodiment, the pull wire assembly machine 100 includes one or more collection ports 136, 138. The assembled pull wire assemblies 10 may be collected from the collection ports 136, 138. **In** the illustrated embodiment, the collection ports 136, 138 are located at the front 120. Other locations are possible in alternative embodiments. **In** an exemplary embodiment, the pull wire assemblies 10 are sorted within the pull wire assembly machine 100 for collection at the different collection ports 136, 138. For example, the pull wire assemblies 10 may be quality checked after assembly to determine if the assembled pull wire assembly 10 passes or fails a quality threshold. The pull wire assemblies 10 may be sorted into good/not good piles for retrieval by the operator. For example, the passing or good pull wire assemblies 10 may be collected through the first collection port 136 whereas the failing or not good pull wire assemblies 10 may be collected through the second collection port 138. Other sorting criteria may be used in alternative embodiments, such as sorting the pull wire assemblies 10 based on size and/or length. Greater or fewer collection ports 136, 138 may be used in alternative embodiments based on the sorting criteria. In an exemplary embodiment, the pull wire assemblies 10 may be manually collected by an operator from the collection ports 136, 138. For example, the removal system 500 may allow removal of the pull wire assemblies 10 through the collection ports 136, 138. In other various embodiments, the pull wire assemblies 10 may be automatically collected, such as by a robot, for transport to in other processing or assembly machine.

In an exemplary embodiment, the pull wire assembly machine 100 includes a control system 150 for controlling one or more operations of the pull wire assembly machine 100. The control system 150 includes one or more processors for performing operational control of the various components of the systems of the pull wire assembly machine 100. The control system 150 may provide electronic control, hydraulic control, pneumatic control, or other types of controls for the components of the systems. In an exemplary embodiment, the control system 150 includes one or more sensors for controlling the components of the systems. For example, the control system 150 may include position sensors. The control system 150 may include one or more communication modules for communicating between the various components and/or for communicating with a remote device, such as a remote computer or a handheld device used by the operator controlling the pull wire assembly machine 100. The communication module may communicate by a wired connection or a wireless connection.

In an exemplary embodiment, the control system 150 includes a vision system 160 for controlling the components of the systems. For example, the vision system 160 may include one or more cameras for imaging the components and/or the pull wire 20 and/or the pull ring 30 and/or the pull wire assembly 10. The vision system 160 may be used to position the pull wire 20 relative to the pull ring 30 at the assembly zone 102. The vision system 160 may be used to control the laser welding of the pull wire 20 to the pull ring 30. The vision system 160 may be used for quality inspection of the pull wire assembly 10.

In an exemplary embodiment, the control system 150 includes a user interface 170 for the operator. In various embodiments, the user interface 170 includes a display 172, one or more inputs 174, and one or more outputs 176. The display 172 may show images from the vision system and 60. The display 172 may show control functions available to the operator for control of the pull wire assembly machine 100. The inputs 174 may include a mouse, a keypad, buttons, dials, a touchscreen, or other types of inputs. The inputs 174 may include a USB port, an ethernet port, or another type of data port. The outputs 176 may include lights, speakers, indicators, or other types of outputs for the operator. The outputs 176 may relate to the operational status of the pull wire assembly machine 100. The outputs 176 may be provided on the display 172 or may be separate outputs.

Figure 5 is a perspective view of the pull wire assembly 10 in accordance with an exemplary embodiment. Figure 6 is a top view of the pull wire assembly 10 in accordance with an exemplary embodiment. Figure 7 is an enlarged view of a portion of the pull wire assembly 10 in accordance with an exemplary embodiment showing the pull wire 20 laser welded to the pull ring 30. **In** the illustrated embodiment, the pull wire assembly 10 includes a single pull wire 20. However, the pull wire assembly 10 may include multiple pull wires 20 in alternative embodiments.

In an exemplary embodiment, the pull wire 20 is a stainless steel wire having a predetermined length and diameter for a particular application. For example, the pull wire 20 may have a length of 50 inches (127 cm). The pull wire 20 may be longer or shorter in alternative embodiments, such as having a length of approximately 72 inches (183 cm). The pull wire 20 may have a diameter of .007 inches (.1778mm). However, the pull wire 20 may have a wider or narrower diameter in alternative embodiments. The pull wire 20 may be manufactured from a different material in alternative embodiments. The pull wire 20 may be coated or plated in various embodiments.

In an exemplary embodiment, the pull ring 30 is a stainless steel ring having a predetermined length and diameter for a particular application. The pull ring 30 extends between a front 32 and a rear 34. The pull ring 30 has an inner surface 36 and an outer surface 38. In various embodiments, the pull ring 30 has a length between the front 32 and the rear 34 of 0.1 inches (.254 cm). In various embodiments, the pull ring 30 has an outer diameter of .198 inches (.501 cm) and an inner diameter of .178 inches (.452 cm). However, the pull ring 30 may have other inner and outer diameters in alternative embodiments. The pull ring 30 may be manufactured from a different material in alternative embodiments. The pull ring 30 may be coated or plated in various embodiments. In an exemplary embodiment, the pull ring 30 includes a slot 40. The slot 40 is configured to receive the pull wire 20. The slot 40 may have a width approximately equal to the diameter of the pull wire 20. In the illustrated embodiment, the slot 40 is open at the rear 34 to receive the pull wire 20, which extends rearward from the pull ring 30. The pull wire 20 is laser welded to the pull ring 30 in the slot 40 at a weld joint 42.

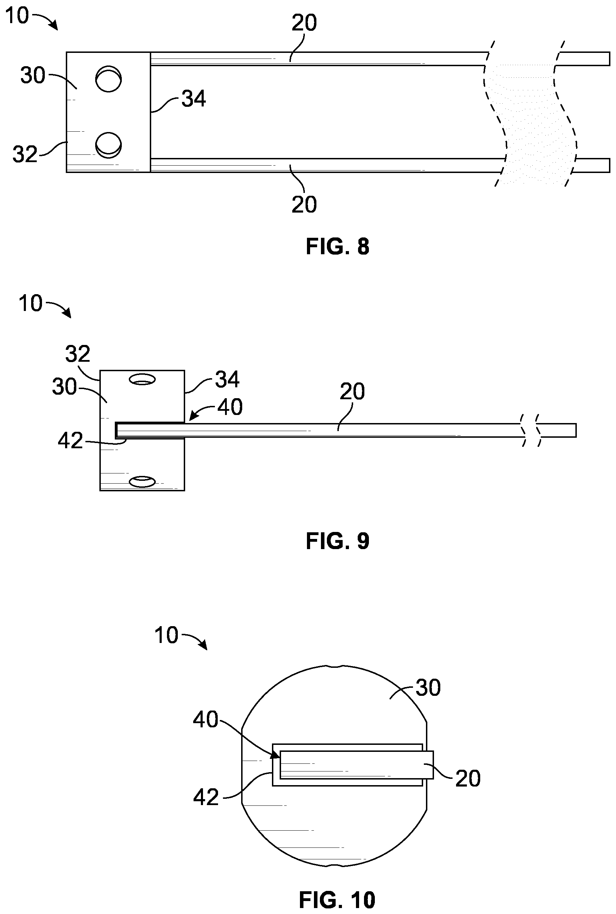

Figure 8 is a top view of the pull wire assembly 10 in accordance with an exemplary embodiment. Figure 9 is a side view of the pull wire assembly 10 in accordance with an exemplary embodiment. Figure 10 is an enlarged view of a portion of the pull wire assembly 10 in accordance with an exemplary embodiment showing the pull wire 20 laser welded to the pull ring 30. In the illustrated embodiment, the pull wire assembly 10 includes a pair of the pull wires 20 coupled to the pull ring 30.

In an exemplary embodiment, the pull wires 20 are stainless steel wires. In an exemplary embodiment, the pull ring 30 is a stainless steel ring extending between the front 32 and the rear 34. In the illustrated embodiment, the pull ring 30 includes a pair of the slots 40 on opposite sides of the pull ring 30. Each slot 40 is configured to receive the corresponding pull wire 20. The pull wires 20 are laser welded to the pull ring 30 in the slots 40 to form a weld joint 42.

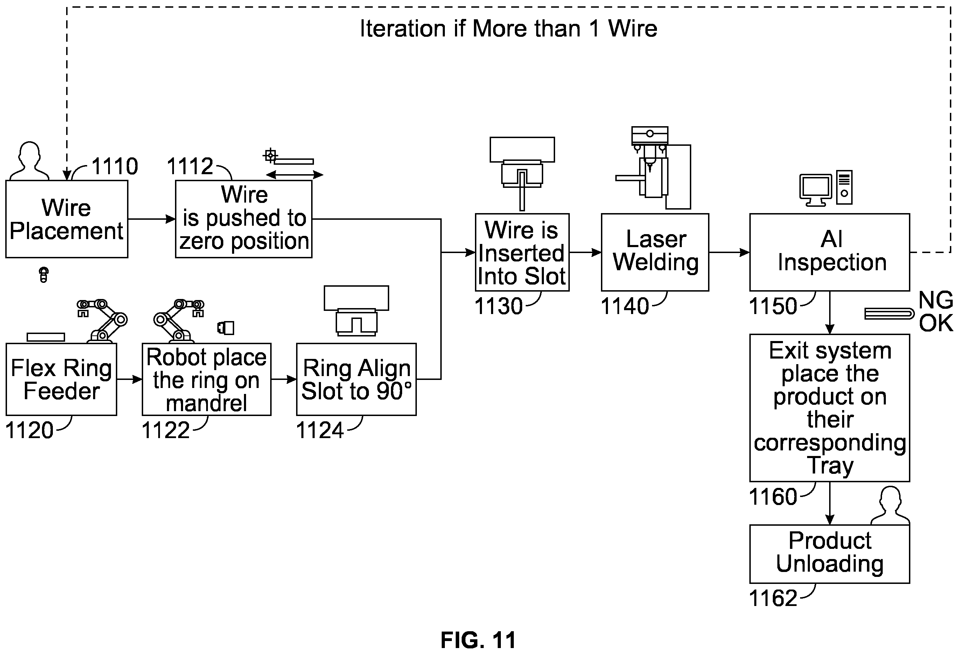

Figure 11 illustrates an assembly method for assembling the pull wire assembly 10 in accordance with an exemplary embodiment. The assembly method may be performed using the pull wire assembly machine 100 shown in Figure 1.

At step 1110, the method includes placing the pull wire 20 into the pull wire assembly machine 100. The pull wire 20 may be manually loaded into the pull wire assembly machine 100 by the operator, such as through the wire load port 134 at the front of the cabinet 110. At step 1112, the method includes pushing the pull wire 20 to a home position. The wire placement system 200 may be used to position the pull wire 20 at the home position.

At step 1120, the method includes picking the pull ring 30 from a ring feeder device. The pull ring 30 may be picked using the ring placement system 300. For example, a multi-axis robot may be used to pick up the pull ring 30. At step 1122, the method includes placing the pull ring 30. The pull ring 30 may be placed by the multi-axis robot. The pull ring 30 may be placed on a mandrel used to support the pull ring 30 at the assembly zone during the assembly process. At step 1124, the method includes orienting the pull ring 30 in the assembly zone. The pull ring 30 may be rotated to a predetermined position in the assembly zone. For example, the slot 40 may be aligned with the pull wire 20 at the home position. In various embodiments, the slot 40 of the pull ring 30 may be oriented in an upright or 90° position.

At step 1130, the method includes inserting the pull wire 20 into the slot 40 of the pull ring 30. The wire placement system 200 may be used to advance the pull wire 20 into the slot 40 of the pull ring 30.

At step 1140, the method includes laser welding the pull wire 20 to the pull ring 30. The laser weld system 400 may be used to laser weld the pull wire 20 to the pull ring 30.

At step 1150, the method includes a quality inspection of the pull wire assembly 10. For example, the laser weld joint between the pull wire 20 and the pull ring 30 may be inspected by the vision system to determine if the laser weld passes or fails the quality inspection.

At step 1160, the method includes removing the pull wire assembly 10 from the assembly zone to the removal zone. The pull wire assembly 10 is removed from the assembly zone to allow assembly of the next pull wire assembly 10. For example, the removal system 500 may be used to remove the pull wire assembly 10 from the assembly zone. The pull wire assembly 10 may be removed based on the quality inspection. For example, the pull wire assembly 10 may be sorted into different trays based on the pull wire assembly 10 passing or failing the quality inspection. At step 1162, the method includes unloading the product from the pull wire assembly machine 100. The pull wire assemblies 10 may be manually unloaded from the removal zone, such as from the respective trays of the removal system 500.

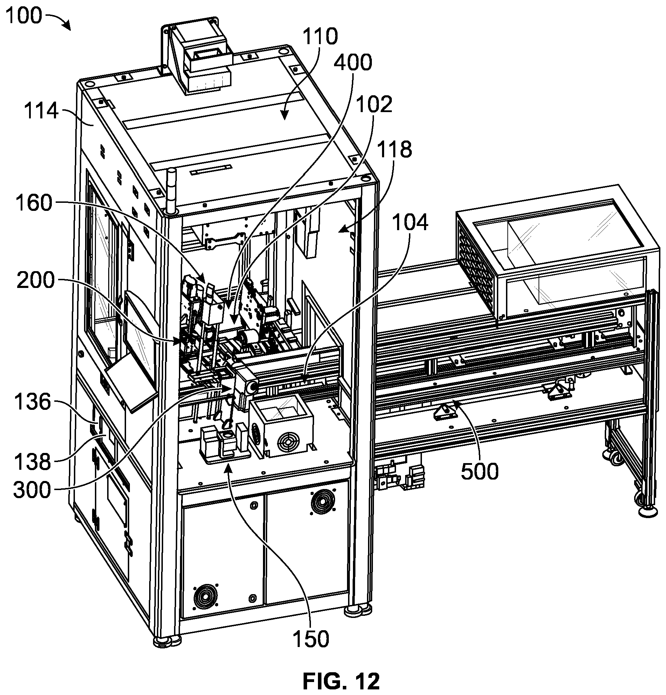

Figure 12 is a perspective view of the pull wire assembly machine 100 in accordance with an exemplary embodiment. Figure 12 has one of the side panels 114 removed to illustrate the components of the systems of the pull wire assembly machine 100 in the compartment 118 of the cabinet 110. The pull wire assembly machine 100 includes the wire placement system 200, the ring placement system 300, the laser weld system 400, and the removal system 500. The wire placement system 200 is used to place the pull wire 20 in the assembly zone 102 for connection to the pull ring 30. The ring placement system 300 is used to position the pull ring 30 in the assembly zone 102 for connection to the pull wire 20. The laser weld system 400 is used to laser weld the pull wire 20 to the pull ring 30. The removal system 500 is used to move the assembled pull wire assembly 10 to the removal zone 102, such as for removal from the machine. The removal system 500 may sort the assembled pull wire assemblies 10, such as based on a quality assessment of the pull wire assemblies 10.

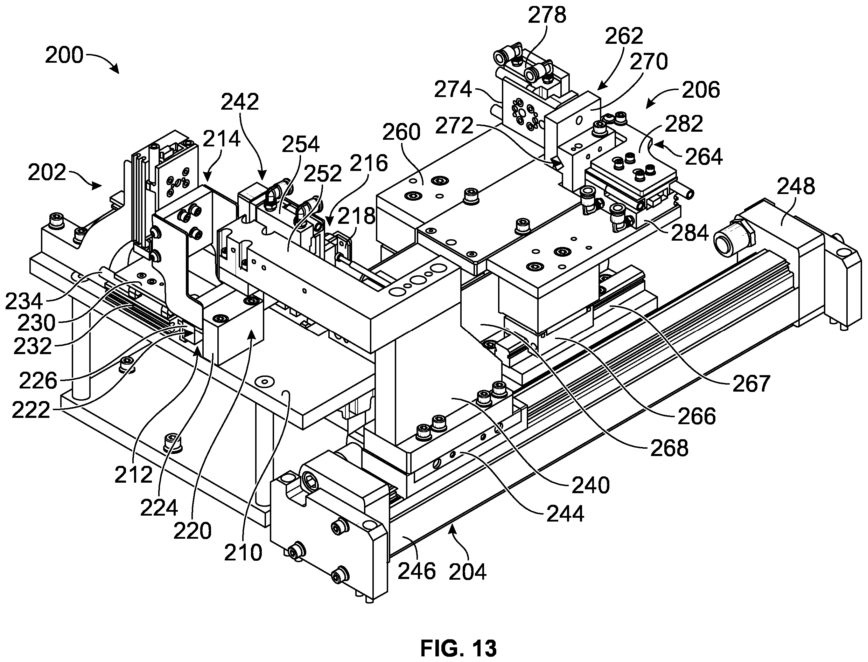

In an exemplary embodiment, the wire placement system 200 includes a wire guide module 202, a wire pull module 204, and a wire insertion module 206 (each shown in Figure 13). The wire guide module 202 is configured to guide the pull wire 20 into the pull wire assembly machine 100, such as to an initial or home position. The wire pull module 204 is configured to pull or advance the pull wire 20 from the initial position to an advanced position. The wire insertion module 206 is configured insert the end of the pull wire 20 into the pull ring 30 at the assembly zone 102. The control system 150 controls the components of the wire guide module 202, the wire pull module 204, and the wire insertion module 206 during the assembly process. The vision system 160 may be used to control the components of the modules 202, 204, 206.

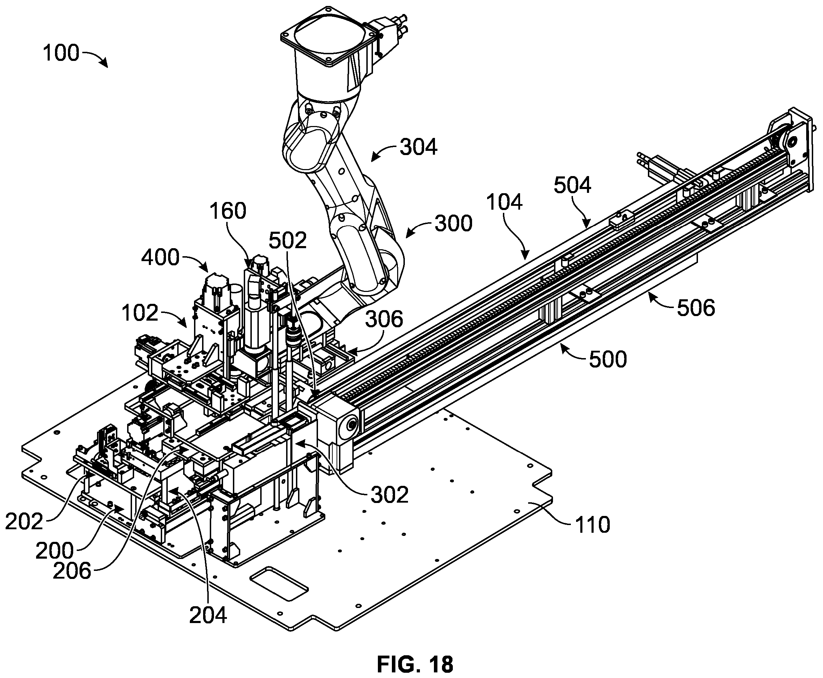

In an exemplary embodiment, the ring placement system 300 includes a ring feeder 302, a ring manipulator 304, and a ring holder 306 (each shown in Figure 18). The ring feeder 302 supplies the pull rings 30 to the ring placement system 300. For example, the ring feeder 302 may hold a plurality of the pull rings 30 on a tray or platform configured to be picked up by the ring manipulator 304. The ring manipulator 304 is used to pick and place the pull rings 30. For example, the ring manipulator 304 may pick up individual pull rings 30 from the ring feeder 302 and move the pull ring 30 to the assembly zone 102. The ring manipulator 304 may place the pull ring 30 on the ring holder 306. **In** various embodiments, the ring manipulator 304 is a multi-axis robot, such as a four axis robot or a six axis robot, that is movable in three-dimensional space. The ring holder 306 is located at the assembly zone 102. The ring holder 306 is configured to hold the pull ring 30 during assembly with the pull wire 20.

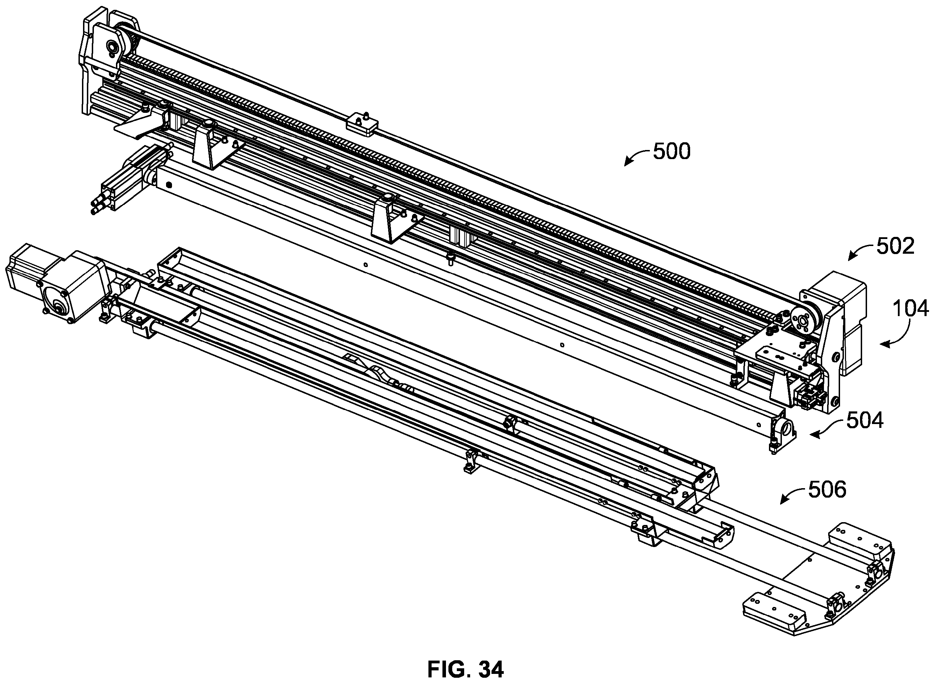

In an exemplary embodiment, the removal system 500 includes a transport device 502, a sorting tray assembly 504, and a storage tray assembly 506 (each shown in Figure 34). The transport device 502 is configured to gather the assembled pull wire assembly 10 and transport the pull wire assembly 10 from the assembly zone 102 to the removal zone 104. The transport device 502 releases the pull wire assembly 10 into the sorting tray assembly 504. The sorting tray assembly 504 is used to sort the pull wire assembly 10 into the storage tray assembly 506. For example, the pull wire assemblies 10 may be sorted based on a quality assessment, such as based on a determination that the pull wire assembly 10 passes or fails a quality threshold. The pull wire assemblies 10 may be sorted based on size of the pull ring 30 and/or length of the pull wire 20. The pull wire assemblies 10 are removed into the storage tray assembly 506. In an exemplary embodiment, multiple pull wire assemblies 10 may be stored in the storage tray assembly 506 prior to removal from the pull wire assembly machine 100. The removal system 500 may allow removal of the pull wire assemblies 10 by opening the collection ports 136, 138 to access the storage tray assembly 506 and remove the pull wire assemblies 10 from the storage tray assembly 506, such as for discarding the defective pull wire assemblies or for further processing of the passing pull wire assemblies.

The pull wire assembly machine 100 automates the assembly process of the pull wire assemblies 10. For example, the pull wire assembly machine 100 automatically positions the pull wire 20 and the pull ring 30 for the laser welding process. The pull wire assembly machine 100 automates the laser welding process. The pull wire assembly machine 100 uses imaging to assess the quality of the laser weld between the pull wire 20 and the pull ring 30. In an exemplary embodiment, the pull wire assembly machine 100 supports the pull wire assemblies 10 based on the quality assessment for the operator to gather the pull wire assemblies 10 for further processing. The assembly process for the pull wire assemblies 10 is repeatable and may be performed at high quality with limited waste by automating the assembly process. The assembly process may be completed quickly to increase throughput of the product. For example, the automated assembly process may be completed in under 20 seconds for a single wire assembly or under 40 seconds for a dual wire assembly compared to the manual assembly process, which typically takes approximately 50 seconds for a single wire assembly or 75 seconds for a dual wire assembly.

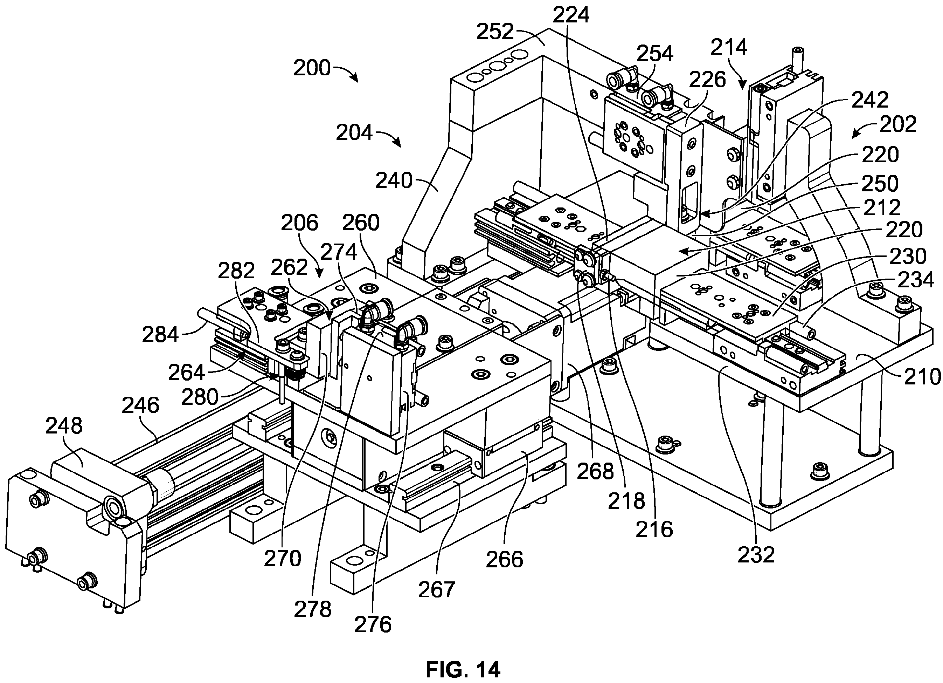

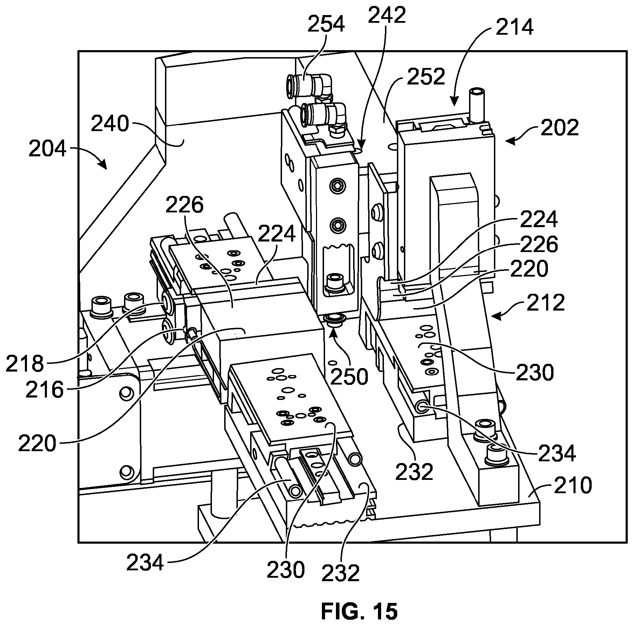

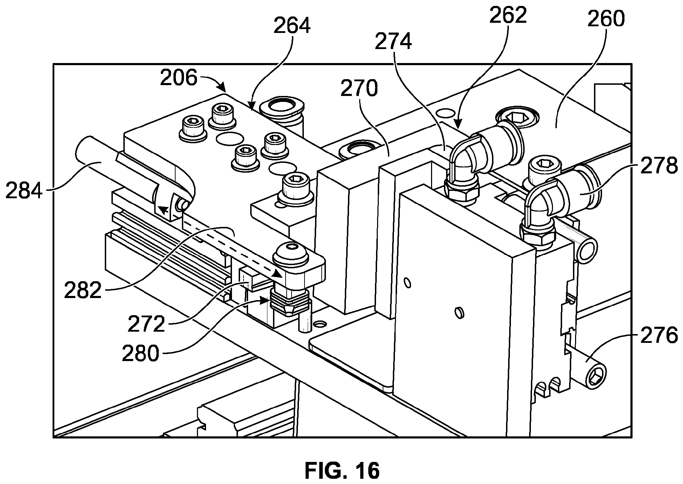

Figure 13 is a front perspective view of the wire placement system 200 in accordance with an exemplary embodiment. Figure 14 is a rear perspective view of the wire placement system 200 in accordance with an exemplary embodiment. Figure 15 is an enlarged view of a portion of the wire placement system 200 in accordance with an exemplary embodiment. Figure 16 is an enlarged view of a portion of the wire placement system 200 in accordance with an exemplary embodiment. The wire placement system 200 includes the wire guide module 202, the wire pull module 204, and the wire insertion module 206. Figure 15 shows a portion of the wire guide module 202 and a portion of the wire pull module 204. Figure 16 shows a portion of the wire insertion module 206.

The wire guide module 202 includes a guide module platform 210 and a guide module guide device 212 mounted to the guide module platform 210. The guide module platform 210 may be mounted to the cabinet 110, such as to the frame 112 or another component of the cabinet 110. The guide module platform 210 may be stationary within the compartment of the cabinet 110. In various embodiments, the guide module platform 210 may be positioned at the front of the cabinet 110 to receive the pull wire 20. The guide module guide device 212 is used to guide the pull wire 20 into the pull wire assembly machine 100, such as to an initial or home position. Optionally, the pull wire 20 may be manually loaded into the guide module guide device 212, such as through the wire load port 134 at the front of the cabinet 110. The wire pull module 204 may gather the pull wire 20 from the initial position to advance the pull wire 20 to the advanced position.

In an exemplary embodiment, the wire guide module 202 includes a wire pusher 214 mounted to the guide module platform 210 to push the pull wire 20 out of the guide module guide device 212, such as after the pull wire 20 is advanced to the advanced position by the wire pull module 204. The wire pusher 214 may be electronically actuated, hydraulically actuated, or pneumatically actuated. The wire pusher 214 may be operably connected to the control system 150 to control advancing or retracting of the wire pusher 214, such as coordinated with other assembly processes of the pull wire assembly machine 100. The wire pusher 214 is movable relative to the guide module guide device 212. For example, the wire pusher 214 may be moved in a vertical direction, such as to push the pull wire 20 in a downward direction out of the guide module guide device 212.

In an exemplary embodiment, the wire guide module 202 includes a position sensor 216 used to sense a position of the pull wire 20 in the wire guide module 202. For example, the position sensor 216 is used to sense the pull wire 20 at the initial or home position. In various embodiments, the pull wire 20 is manually inserted into the pull wire assembly machine 100 until the end of the pull wire 20 is at the initial or home position, which is sensed by the position sensor 216. The wire guide module 202 may include a load stop 218 to stop loading of the pull wire 20 into the guide module guide device 212. Signals from the position sensor 216 may be used to control operation of other components of the pull wire assembly machine 100, such as the wire pull module 204.

In an exemplary embodiment, the guide module guide device 212 includes one or more guide blocks 220 mounted to the guide module platform 210. Each guide block 220 includes a guide channel 222 that receives the pull wire 20. The pull wire 20 is guided by the guide block 220 through the guide channel 222. The guide channel 222 is sized and shaped to receive the pull wire 20. Optionally, the guide channel 222 may include a chamfered opening to funnel the pull wire 20 into the guide channel 222.

In an exemplary embodiment, the guide blocks 220 may be opened and closed to receive and release the pull wire 20. For example, each guide block 220 of the guide module guide device 212 includes a first guide member 224 and a second guide member 226 on opposite sides of the guide channel 222. The first and second guide members 224, 226 may be movable relative to each other. For example, one or both of the guide members 224, 226 may be movable relative to the guide module platform 210. The guide members 224, 226 may be spread apart or opened to release the pull wire 20 from the guide channel 222. The guide members 224, 226 are pressed together to form the guide channel 222 therebetween.

In an exemplary embodiment, the guide blocks 220 include a slide 230 slidably coupled to a rail 232 and an actuator 234 operably coupled to the slide 230 to move the slide 230 on the rail 232. The rail 232 may include a track to guide movement of the slide 230 on the rail 232. The slide 230 may be movable in a linear direction, such as in a direction perpendicular to the axis of the pull wire (for example, side to side). The guide members 224, 226 are coupled to the corresponding slide 230 and are movable with the slide 230 to open and close the guide block 220. In an exemplary embodiment, the pull wire 20 is initially loaded into the wire guide module 202 with the guide blocks 220 in the closed positions. The guide blocks 220 may be moved to the open positions after the wire pull module 204 picks up the pull wire 20 to advance the pull wire 20 in the advancing direction. The actuator 234 may be electrically actuated, hydraulically actuated, or pneumatically actuated. The actuator 234 is operably coupled to the control system 150 to control opening and closing of the guide blocks 220.

The wire pull module 204 includes a pull module platform 240 and a pull module gripper assembly 242 mounted to the pull module platform 240. The pull module gripper assembly 242 is configured to grip the pull wire 20 and move the pull wire 20 within the wire placement system 200. In an exemplary embodiment, the pull module platform 240 is movable within the compartment of the cabinet 110 to advance the pull wire 20 from the initial or home position to an advanced position. For example, the pull module platform 240 includes a slide 244 coupled to a rail 246 and an actuator 248 operably coupled to the slide 244 to move the slide 244 on the rail 246. The rail 246 may include a track to guide movement of the slide 244 on the rail 246. The slide 244 may be movable in a linear direction, such as a direction parallel to the axis of the pull wire 20 (for example, front to rear). The actuator 248 may be electrically actuated, hydraulically actuated, or pneumatically actuated. The actuator 248 is operably coupled to the control system 150 to control movement of the pull module gripper assembly 242 in the advancing direction.

The pull module gripper assembly 242 includes a wire gripper 250 mounted to a support arm 252. The support arm 252 may be mounted to the pull module platform 240 and is movable in the linear direction with the pull module platform 240. The support arm 252 may be manufactured from one or more pieces, such as metal plates. The wire gripper 250 includes one or more gripper elements, such as gripper fingers, configured to grip the pull wire 20 and move the pull wire 20 within the wire placement system 200. In an exemplary embodiment, the pull module gripper assembly 242 includes an actuator 254 operably coupled to the wire gripper 250 to close and open the wire gripper 250, such as to grip and release the pull wire 20, respectively. The actuator 254 may be electrically actuated, hydraulically actuated, or pneumatically actuated. The actuator 254 is operably coupled to the control system 150 to control opening and closing of the wire gripper 250.

The wire insertion module 206 includes an insertion module platform 260, and insertion module guide device 262, and an insertion module gripper assembly 264. The insertion module gripper assembly 264 is configured to grip the pull wire 20 and move the pull wire 20 within the wire placement system 200, such as for inserting the pull wire 20 into the pull ring 30 during assembly. For example, the insertion module gripper assembly 264 may move the pull wire 20 from the advanced position to an insertion position, wherein the end of the pull wire 20 is inserted into the slot 40 the pull ring 30.

In an exemplary embodiment, the pull module platform 260 is movable within the compartment of the cabinet 110 to advance the pull wire 20 from the advanced position to the inserted position. For example, the pull module platform 260 includes a slide 266 coupled to a rail 267 and an actuator 268 operably coupled to the slide 266 to move the slide 266 on the rail 267. The rail 267 may include a track to guide movement of the slide 266 on the rail 267. Optionally, multiple slides 266 and rails 267 may be provided, such as on opposite sides of the pull module platform 260. The slide 266 may be movable in a linear direction, such as a direction parallel to the axis of the pull wire 20 (for example, front to rear). The actuator 268 may be electrically actuated, hydraulically actuated, or pneumatically actuated. The actuator 268 is operably coupled to the control system 150 to control insertion of the pull wire 20 into the pull ring 30.

The insertion module guide device 262 is located proximate to the assembly zone 102. The insertion module guide device 262 is used to guide positioning of the end of the pull wire 20 at the assembly zone 102. For example, the insertion module guide device 262 may align the end of the pull wire 20 with the pull ring 30. The pull wire 20 is loaded into the insertion module guide device 262 by the wire pull module 204. For example, the pull module gripper assembly 242 is configured to load the end of the pull wire 20 into the insertion module guide device 262 as the buyer pull module 204 is moved in the advancing direction.

In an exemplary embodiment, the insertion module guide device 262 includes a guide block 270 mounted to the insertion module platform 260. The guide block 270 includes a guide channel 272 that receives the pull wire 20. The pull wire 20 is guided by the guide block 270 through the guide channel 272. The guide channel 272 is sized and shaped to receive the pull wire 20. Optionally, the guide channel 272 may include a chamfered opening to funnel the pull wire 20 into the guide channel 272.

In an exemplary embodiment, the guide block 270 may be opened and closed to receive and release the pull wire 20, such as similar to the guide block 220. In alternative embodiments, the guide block 270 does not open and close, but rather is a unitary structure, such as being molded, cast, or formed from a single piece of material.

**In** an exemplary embodiment, the guide block 270 is movably coupled to the insertion module platform 260. For example, the guide block 270 may be moved relative to the insertion module platform 260 to align the pull wire 20 with the pull ring 30. **In** various embodiments, the guide block 270 may be moved vertically and/or horizontally. The guide block 270 includes a slide 274 slidably coupled to a rail 276 and an actuator 278 operably coupled to the slide 274 to move the slide 274 on the rail 276. The rail 276 may include a track to guide movement of the slide 274 on the rail 276. The slide 274 may be movable in a linear direction, such as in a direction perpendicular to the axis of the pull wire (for example, side to side). The actuator 278 may be electrically actuated, hydraulically actuated, or pneumatically actuated. The actuator 278 is operably coupled to the control system 150 to control movement of the guide block 270, such as to align the pull wire 20 with the pull ring 30.

The insertion module gripper assembly 264 is configured to grip the pull wire 20 and move the pull wire 20 within the wire placement system 200. For example, the insertion module gripper assembly 264 is used to advance the pull wire 20 into the assembly zone 102 to insert the pull wire 20 into the slot 40 in the pull ring 30. In an exemplary embodiment, the insertion module gripper assembly 264 includes a wire gripper 280 mounted to a support arm 282. The support arm 282 may be mounted to the insertion module platform 260 and is movable in the linear advancing direction with the insertion module platform 260. The support arm 282 may be manufactured from one or more pieces, such as metal plates. The wire gripper 280 includes one or more gripper elements, such as gripper fingers, configured to grip the pull wire 20 and move the pull wire 20 within the wire placement system 200. In an exemplary embodiment, the insertion module gripper assembly 264 includes an actuator 284 operably coupled to the wire gripper 280 to close and open the wire gripper 280, such as to grip and release the pull wire 20, respectively. The actuator 284 may be electrically actuated, hydraulically actuated, or pneumatically actuated. The actuator 284 is operably coupled to the control system 150 to control opening and closing of the wire gripper 280.

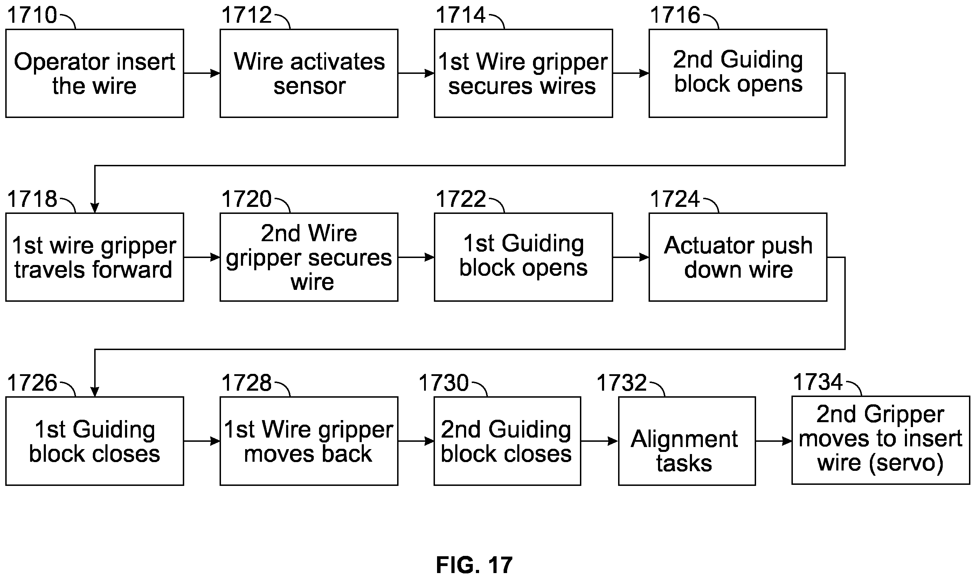

With additional reference to Figure 17, which shows a method of loading the pull wire 20 into the assembly zone 102 of the pull wire assembly machine 100. At 1710 the method includes the operator inserting the wire into the pull wire assembly machine 100. For example, the pull wire 20 may be manually loaded into the cabinet 110 through the wire load port 134. The wire guide module 202 of the wire placement system 200 is used to guide the pull wire 20 into an initial or home position within the cabinet 110. As the pull wire 20 is initially loaded into the wire placement system 200, the end of the pull wire 20 is loaded into the guide channels 222 of the guide blocks 220 of the guide module guide device 212. For example, the pull wire 20 may pass through the first or front guide block 220, through an open space, into the second or rear guide block 220. The wire gripper 250 of the pull module gripper assembly 242 is located in the open space between the front and rear guide blocks 220. At the end of the rear guide block 220, the end of the pull wire 20 may engage the load stop 218.

At 1712, the method includes activating the position sensor 216. For example, when the end of the pull wire 20 is at the load stop 218, the position sensor 216 senses the location of the pull wire 20 at the initial or home position. At 1714, the method includes securing the pull wire 20 with the wire gripper 250. For example, the position sensor 216 may activate the pull module gripper assembly 242 causing the actuator 254 to close the wire gripper 250 to grip the pull wire 20. At 1716, the method includes opening the rear guide block 220. For example, the actuator 234 may be operated to spread the first and second guide members 224, 226 of the rear guide block 220 apart from each other to provide a space to allow the wire gripper 250 to move in the advancing direction through the guide block 220. At 1718, the method includes advancing the wire gripper in the advancing direction to the advanced position. The wire gripper 250 travels rearward towards the wire insertion module 206. The wire gripper 250 loads the end of the pull wire 20 into the insertion module guide device 262 as the wire pull module 204 is advanced. For example, the end of the pull wire 20 is loaded into the guide channel 272 in the guide block 270.

At 1720, the wire gripper 280 of the insertion module gripper assembly 264 is activated to grip and secure the pull wire 20 in the wire insertion module 206. For example, the actuator 284 is operated to close the wire gripper 280 to grip the end of the pull wire 20 and hold the pull wire 20 in the insertion module guide device 262. At 1722, the first or front guide block 220 of the wire guide module 202 is opened to release the pull wire 20. For example, the actuator 234 is operated to open the first guide member 224 and/or the second guide member 226. At 1724, the wire pusher 214 is operated to push the pull wire 20 downward out of the guide channel 222 of the front guide block 220. At 1726, the front guide block 220 of the wire guide module 202 is closed to form the guide channel 222 to receive the next pull wire 20. For example, the actuator 234 is operated to close the first guide member 224 and/or the second guide member 226. At 1728, the wire gripper 250 is retracted and moved back to a home position between the front and rear guide blocks 220 of the wire guide module 202. At 1730, the rear guide block 220 of the wire guide module 202 is closed to form the guide channel 222 to receive the next pull wire 20.

At 1732, the wire insertion module 206 is operated to align the pull wire 20 with the pull ring 30. For example, the actuator 278 is operated to position the guide block 270 to align the end of the pull wire 20 with the pull ring 30. The guide block 270 may be moved side to side to align the pull wire 20 with the slot 40 of the pull ring 30. At 1734, the wire insertion module 206 is operated to insert the end of the pull wire 20 into the slot 40 of the pull ring 30. For example, the actuator 268 is operated to advance the pull wire 20 toward the pull ring 30. The end of the pull wire 20 is loaded into the slot 40 in the pull ring 30 at the assembly zone 102. The pull wire 20 may be laser welded to the pull ring 30 when the pull wire 20 is inserted into the slot 40.

Figure 18 is a perspective view of internal components of the systems of the pull wire assembly machine 100 in accordance with an exemplary embodiment. The cabinet 110 (Figure 1) is removed in Figure 18 to illustrate the components of the systems of the pull wire assembly machine 100. The pull wire assembly machine 100 includes the wire placement system 200, the ring placement system 300, the laser weld system 400, and the removal system 500. The wire placement system 200 is used to place the pull wire 20 in the assembly zone 102 for connection to the pull ring 30. The ring placement system 300 is used to position the pull ring 30 in the assembly zone 102 for connection to the pull wire 20. The laser weld system 400 is used to laser weld the pull wire 20 to the pull ring 30. The removal system 500 is used to move the assembled pull wire assembly 10 to the removal zone 102, such as for removal from the machine. The removal system 500 may sort the assembled pull wire assemblies 10, such as based on a quality assessment of the pull wire assemblies 10.

In an exemplary embodiment, the wire placement system 200 includes the wire guide module 202, the wire pull module 204, and the wire insertion module 206. The wire guide module 202 is configured to guide the pull wire 20 into the pull wire assembly machine 100, such as to an initial or home position. The wire pull module 204 is configured to pull or advance the pull wire 20 from the initial position to an advanced position. The wire insertion module 206 is configured insert the end of the pull wire 20 into the pull ring 30 at the assembly zone 102. The control system 150 controls the components of the wire guide module 202, the wire pull module 204, and the wire insertion module 206 during the assembly process. The vision system 160 may be used to control the components of the modules 202, 204, 206.

**In** an exemplary embodiment, the ring placement system 300 includes the ring feeder 302, the ring manipulator 304, and the ring holder 306. The ring feeder 302 supplies the pull rings 30 to the ring placement system 300. For example, the ring feeder 302 may hold a plurality of the pull rings 30 on a tray or platform configured to be picked up by the ring manipulator 304. The ring manipulator 304 is used to pick and place the pull rings 30. For example, the ring manipulator 304 may pick up individual pull rings 30 from the ring feeder 302 and move the pull ring 30 to the assembly zone 102. The ring manipulator 304 may place the pull ring 30 on the ring holder 306. In various embodiments, the ring manipulator 304 is a multi-axis robot, such as a four axis robot or a six axis robot, that is movable in three-dimensional space. The ring holder 306 is located at the assembly zone 102. The ring holder 306 is configured to hold the pull ring 30 during assembly with the pull wire 20.

In an exemplary embodiment, the removal system 500 includes the transport device 502, the sorting tray assembly 504, and the storage tray assembly 506. The transport device 502 is configured to gather the assembled pull wire assembly 10 and transport the pull wire assembly 10 from the assembly zone 102 to the removal zone 104. The transport device 502 releases the pull wire assembly 10 into the sorting tray assembly 504. The sorting tray assembly 504 is used to sort the pull wire assembly 10 into the storage tray assembly 506. For example, the pull wire assemblies 10 may be sorted based on a quality assessment, such as based on a determination that the pull wire assembly 10 passes or fails a quality threshold. The pull wire assemblies 10 may be sorted based on size of the pull ring 30 and/or length of the pull wire 20. The pull wire assemblies 10 are removed into the storage tray assembly 506. In an exemplary embodiment, multiple pull wire assemblies 10 may be stored in the storage tray assembly 506 prior to removal from the pull wire assembly machine 100. The removal system 500 may allow removal of the pull wire assemblies 10 by opening the collection ports 136, 138 to access the storage tray assembly 506 and remove the pull wire assemblies 10 from the storage tray assembly 506.

The pull wire assembly machine 100 automates the assembly process of the pull wire assemblies 10. For example, the pull wire assembly machine 100 automatically positions the pull wire 20 and the pull ring 30 for the laser welding process. The pull wire assembly machine 100 automates the laser welding process. The pull wire assembly machine 100 uses imaging to assess the quality of the laser weld between the pull wire 20 and the pull ring 30. In an exemplary embodiment, the pull wire assembly machine 100 supports the pull wire assemblies 10 based on the quality assessment for the operator to gather the pull wire assemblies 10 for further processing. The assembly process for the pull wire assemblies 10 is repeatable and may be performed at high quality with limited waste by automating the assembly process. The assembly process may be completed quickly to increase throughput of the product. For example, the automated assembly process may be completed in under 20 seconds for a single wire assembly or under 40 seconds for a dual wire assembly compared to the manual assembly process, which typically takes approximately 75 seconds for a single wire assembly or 150 seconds for a dual wire assembly.

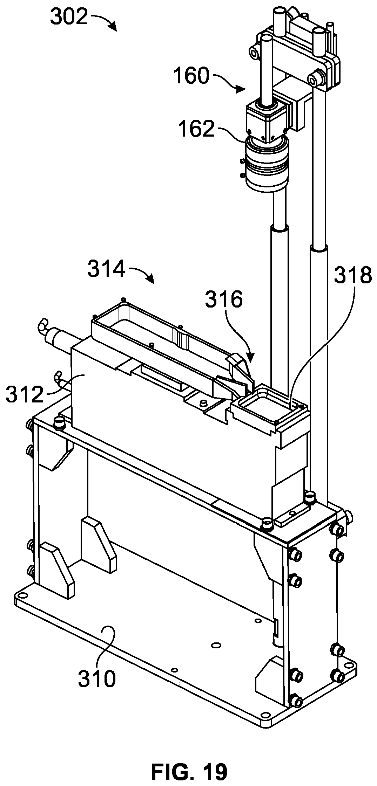

Figure 19 is a perspective view of the ring feeder 302 in accordance with an exemplary embodiment. The ring feeder 302 includes a support platform 310, a feeder device 312 coupled to the support platform 310, and a feeder camera 162 for imaging the feeder device 312. The feeder camera 162 forms part of the vision system 160 used to control operation of other components of the pull wire assembly machine 100, such as the ring manipulator 304, based on images from the feeder camera 162.

The feeder device 312 includes a hopper 314 configured to hold a plurality of the pull rings 30. The hopper 314 includes a funnel 316 at an end of the hopper 314. The pull rings 30 may be dispatched from the hopper 314 through the funnel 316. In an exemplary embodiment, the feeder device 312 includes a feeder tray 318 at the end of the funnel 316. The feeder tray 318 receives the pull rings 30. The pull rings 30 may be picked from the feeder tray 318 by the ring manipulator 304. In an exemplary embodiment, the hopper 314 may be vibrated to advance the pull rings 30 through the funnel 316 onto the feeder tray 318. In an exemplary embodiment, the feeder tray 318 may be vibrated to orient the pull rings 30 on the feeder tray 318. For example, the feeder tray 318 may be vibrated to seat the pull rings 30 on end (for example, seated on the front end of the pull ring) so the pull ring's 30 may be easily picked up by the ring manipulator 304.

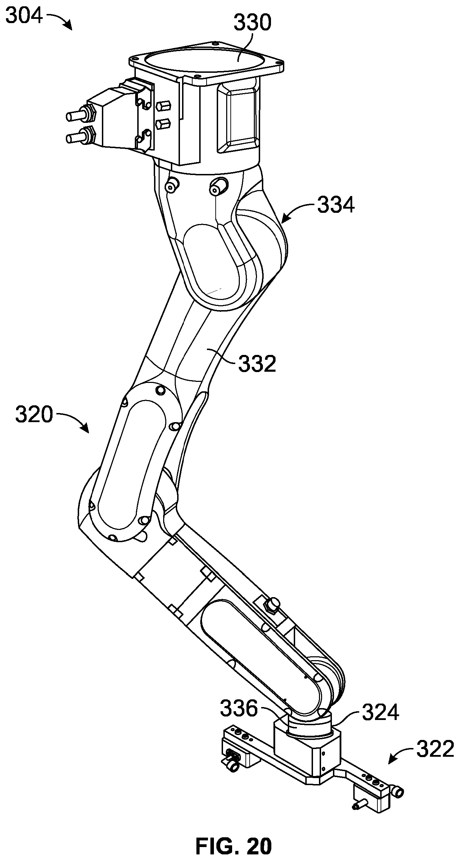

Figure 20 is a perspective view of the ring manipulator 304 in accordance with an exemplary embodiment. In an exemplary embodiment, the ring manipulator 304 includes a robotic arm 320 and an end effector 322 at a distal end 324 of the robotic arm 320. The end effector 322 is used to pick up the pull ring 30 from the ring feeder 302 and placed the pull ring 30 on the ring holder 306. The robotic arm 320 is used to move the end effector 322 and three-dimensional space between the pick and place positions.

The robotic arm 320 includes a mounting base 330 configured be mounted to the cabinet 110, such as to the frame 112. The robotic arm 320 includes link arms 332 attached to the mounting base 330. The link arms 332 are connected to each other at joints 334. The link arms 332 may pivot relative to each other at pivot joints. The link arms 332 may rotate relative to each other at rotary joints. The robotic arm 320 includes a mount 336 at the distal end 324. The end effector 322 is coupled to the mount 336. Optionally, the mount 336 may be rotatable to rotate the end effector 322. In various embodiments, the robotic arm 320 is a four-axis articulated robot having four degrees of freedom. In other various embodiments, the robotic arm 320 is a six-axis articulated robot having six degrees of freedom. The robotic arm 320 may be electrically actuated using electric motors to move the link arms 332 at the joints 334.

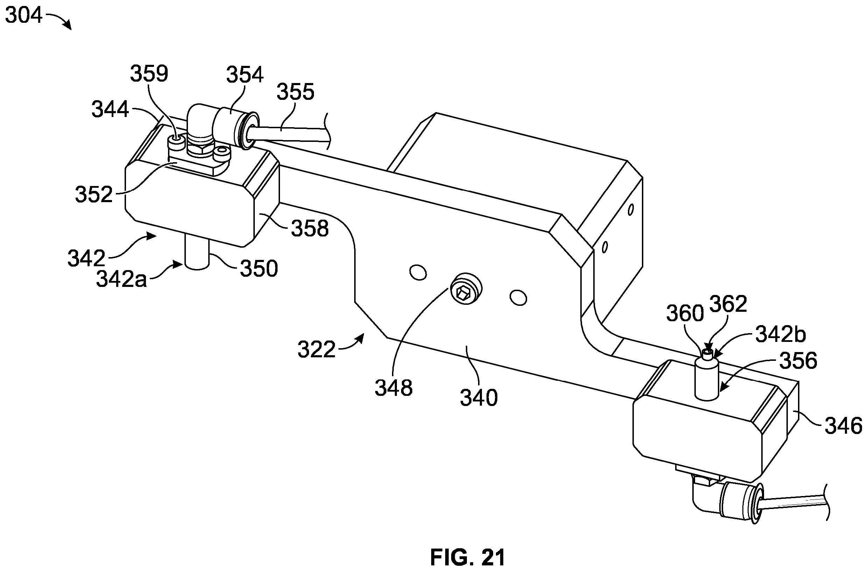

Figure 21 is a perspective view of the end effector 322 of the ring manipulator 304 in accordance with an exemplary embodiment. The end effector 322 includes a mounting bracket 340 and one or more vacuum nozzles 342 coupled to the mounting bracket 340. The mounting bracket 340 is configured to be coupled to the distal end 324 of the robotic arm 320. The vacuum nozzle 342 is configured to pick up the pull ring 30 using vacuum pressure. In the illustrated embodiment, the end effector 322 includes two of the vacuum nozzles 342, such as a large diameter vacuum nozzle 342a and a small diameter vacuum nozzle 342b. However, the end effector 322 may include greater or fewer vacuum nozzles 342 in alternative embodiments. The vacuum nozzles 342a, 342b have different sizes for picking up different size pull rings 30. As such, the same ring manipulator 304 may be used for assembly of different pull wire assemblies and 10 without the need to change out the components of the ring manipulator 304 to accommodate the different sized pull rings 30.

The mounting bracket 340 extends between a first end 344 and a second end 346. The vacuum nozzles 342 are provided at the first and second ends 344, 346 of the mounting bracket 340. The mounting bracket 340 includes a central portion 348 between the first end 344 and the second end 346. The central portion 348 is configured to be mounted to the distal end 324 of the robotic arm 320. Optionally, the central axis of the mounting bracket 340 may be coupled to the robotic arm 320 such that the vacuum nozzles 342 at the first and second ends 344, 346 are spaced equidistant from the central axis of the mounting bracket 340.

Each vacuum nozzle 342 includes a nozzle tube 350 and a nozzle bracket 352 and an end of the nozzle tube 350. A vacuum fitting 354 may be coupled to the nozzle tube 350 and/or the nozzle bracket 352 to connect a vacuum line 355 to the vacuum nozzle 342. The vacuum line 355 pulls negative pressure through the vacuum nozzle 342 to create the vacuum used to pick up the pull ring 30. In an exemplary embodiment, the nozzle tube 350 passes through an opening 356 in a mounting block 358 used to mount the vacuum nozzle 342 to the mounting bracket 340. The nozzle bracket 352 is secured to the mounting block 358 using fasteners 359. Other mounting elements may be used in alternative embodiments to secure the vacuum nozzle 342 to the mounting bracket 340.

In an exemplary embodiment, the vacuum nozzle 342 includes a shroud 360 at the end of the nozzle tube 350 forming a vacuum chamber 362. The vacuum chamber 362 receives the pull ring 30. The vacuum pressure holds the pull ring 30 in the vacuum chamber 362. The shroud 360 is sized and shaped to receive the pull ring 30. In an exemplary embodiment, the different vacuum nozzles 342 different diameter shrouds 360 to receive the different diameter pull rings 30. The shroud 360 may be cylindrical to receive the circular pull ring 30.

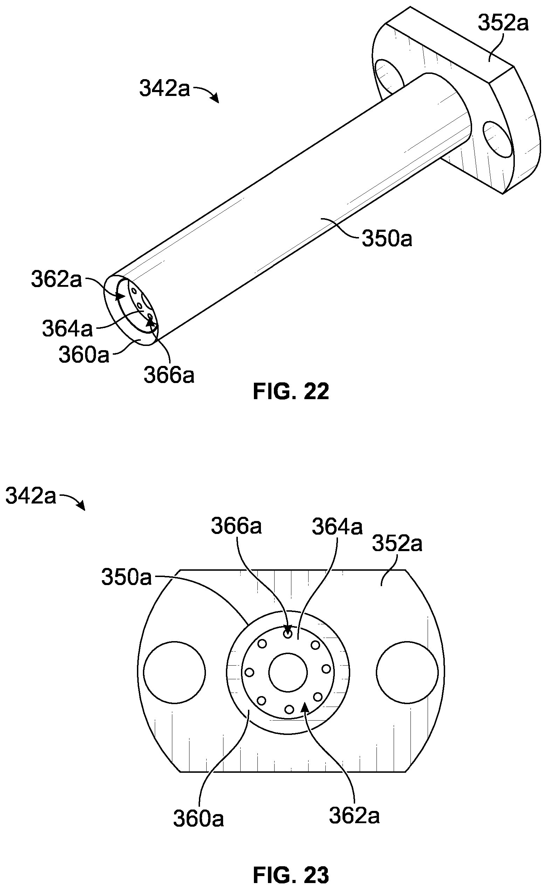

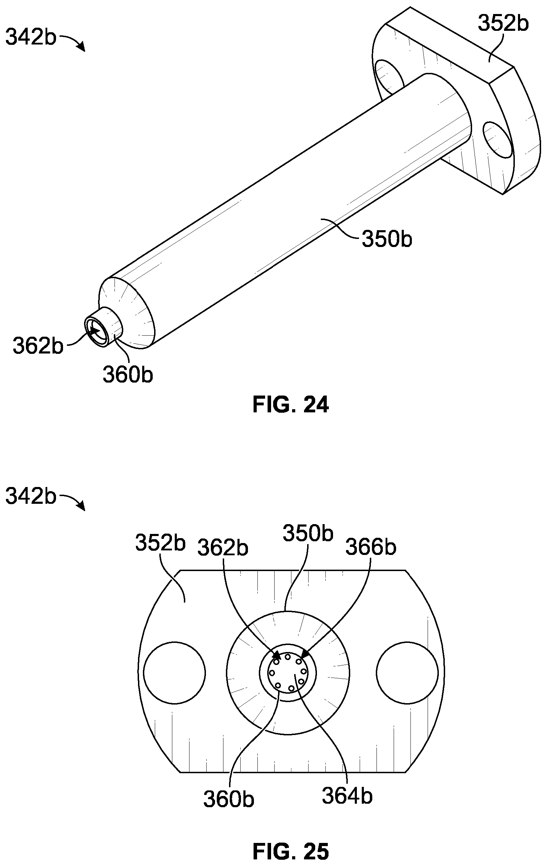

Figure 22 is a perspective view of the large diameter vacuum nozzle 342a in accordance with an exemplary embodiment. Figure 23 is an end view of the large diameter vacuum nozzle 342a in accordance with an exemplary embodiment. Figure 24 is a perspective view of the small diameter vacuum nozzle 342b in accordance with an exemplary embodiment. Figure 25 is an end view of the small diameter vacuum nozzle 342b in accordance with an exemplary embodiment.

The vacuum nozzles 342a, 342b include the nozzle tubes 350a, 350b extending from the nozzle brackets 352a, 352b. The nozzle tubes 350a, 350b may have the same diameters and the nozzle brackets 352a, 352b may have the same dimensions for easy removal and replacement. However, the ends of the vacuum nozzles 342a, 342b are different for use with different sized pull rings. For example, the diameters of the shrouds 360a, 360b are different with the large shroud 360a having a larger diameter and the small shroud 360b have a smaller diameter. The shrouds 360a, 360b are cylindrical. The diameters of the shrouds 360a, 360b correspond to the diameters of the pull rings 30.

The nozzle tubes 350a, 350b have end walls 364a, 364b at the distal ends of the nozzle tubes 350a, 350b located at the interior of the vacuum chambers 362a, 362b. The shrouds 360a, 360b extend forward of the end walls 364a, 364b to form the vacuum chambers 362a, 362b forward of the end walls 364a, 364b. The vacuum nozzles 342a, 342b include vacuum ports 366a, 366b in the end walls 364a, 364b configured to create a vacuum of negative pressure in the vacuum chambers 362a, 362b to hold the pull rings 30 in the vacuum chambers 362a, 362b. In an exemplary embodiment, the vacuum ports 366a, 366b are arranged in a circular pattern. The vacuum ports 366a, 366b are located proximate to the shrouds 360a, 360b. For example, the vacuum ports 366a, 366b may be generally aligned with the pull rings 30 when the pull rings 30 are in the vacuum chambers 362a, 362b.

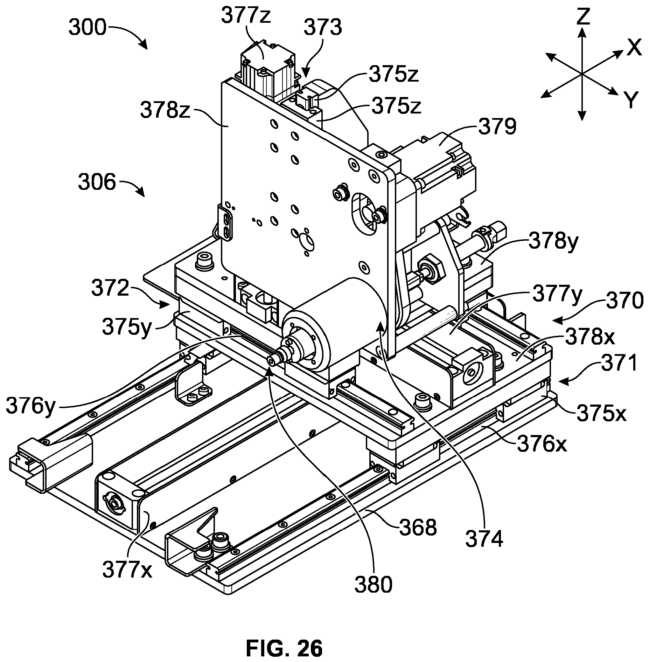

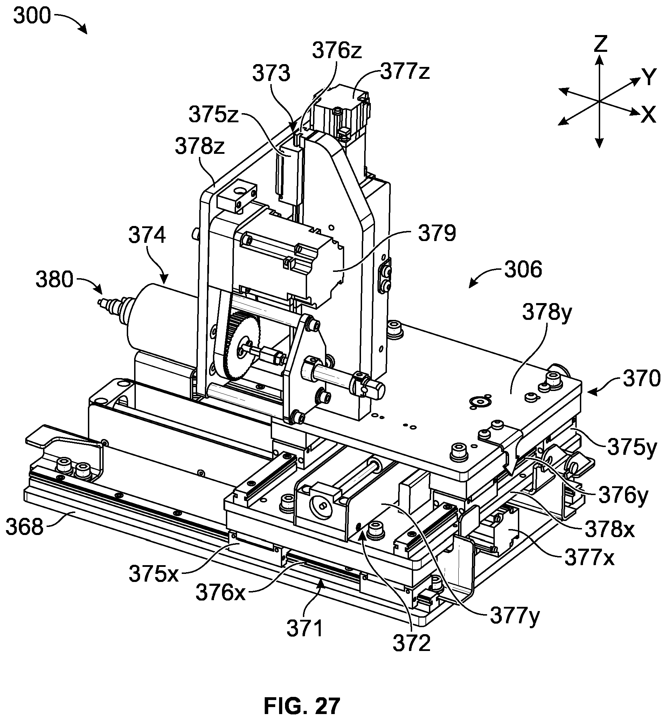

Figure 26 is a front perspective view of the ring holder 306 in accordance with an exemplary embodiment. Figure 27 is a rear perspective view of the ring holder 306 in accordance with an exemplary embodiment. The ring holder 306 includes a ring gantry 370 and a ring mandrel 380 mounted to the ring gantry 370. The ring gantry 370 may be mounted to a base 368, which may be mounted to the cabinet 110 or another component within the cabinet 110. The ring mandrel 380 is used to hold the pull ring 30 at the assembly zone 102. For example, the ring mandrel 380 holds the pull ring 30 for insertion of the pull wire 20 by the wire placement system 200 and a laser welding of the pull wire 20 to the pull ring 30 by the laser weld system 400. The ring gantry 370 is used for positioning the ring mandrel 380. For example, the ring mandrel 380 may be used to control and exposition and/or a wide position and/or a Z position and/or an angular position of the pull ring 30 for loading the pull wire 20 into the slot 40 of the pull ring 30. In an exemplary embodiment, the ring gantry 370 is axially positionable in three-dimensional space (for example, XYZ positioning) and rotatably positionable (for example, 360° rotation).

In an exemplary embodiment, the ring gantry 370 includes an X-positioner 371, a Y-positioner 372, and a Z-positioner 373. The X-positioner 371 is movable in the X direction. The Y-positioner 372 is movable in the Y direction. The Z-positioner 373 is movable in the Z direction. In an exemplary embodiment, the ring gantry 370 includes a rotating mount 374 configured to rotate the ring mandrel 380. For example, the ring mandrel 380 may be mounted to the rotating mount 374. The rotating mount 374 is configured to rotate about a rotation axis to control an angular position of the ring mandrel 380, and thus the pull ring 30 held by the ring mandrel 380.

In an exemplary embodiment, each of the-positioners 371, 372, 373 include a slide 375 (375x, 375y, 375z, respectively) slidably coupled to a rail 376 (376x, 376y, 376z, respectively) and an actuator 377 (377x, 377y, 377z, respectively) operably coupled to the slide 375 to move the slide 375 on the rail 376. The-positioners 371, 372, 373 may each include a platform 378 (378x, 378y, 378z, respectively) used to support other components. The rail 376x of the X-positioner 371 is oriented parallel to the X direction to allow movement of the slide 375x in the X direction. The rail 376y of the Y-positioner 372 is oriented parallel to the Y direction to allow movement of the slide 375y in the Y direction. The rail 376z of the Z-positioner 373 is oriented parallel to the Z direction to allow movement of the slide 375z in the Z direction. In the illustrated embodiment, the X-positioner 371 is mounted to the base 368, the Y-positioner 372 is mounted to the platform 378x of the X-positioner 371, the Z-positioner 373 is mounted to the platform 378y of the Y-positioner 372, and the rotating mount 374 is mounted to the platform 378z of the Z-positioner 373. Other arrangements are possible in alternative embodiments. The actuators 377 may be electrically actuated, hydraulically actuated, or pneumatically actuated. The actuators 377 are operably coupled to the control system 150 to control positioning of the components, such as for positioning the ring mandrel 380 to align the slot 40 of the pull ring 30 with the pull wire 20. The actuators 377 may be controlled based on signals or inputs from the vision system 160, which may be used to align the pull wire 20 with the pull ring 30.

In an exemplary embodiment, the rotating mount 374 includes an actuator 379 to control the angular position of the rotating mount 374. The actuator 379 is operated to rotate the rotating mount 374. The actuator 379 may be directly coupled to the rotating mount 374. In other various embodiments, a linking element, such as a pulley or gear drive is used to couple the actuator 379 to the rotating mount 374.

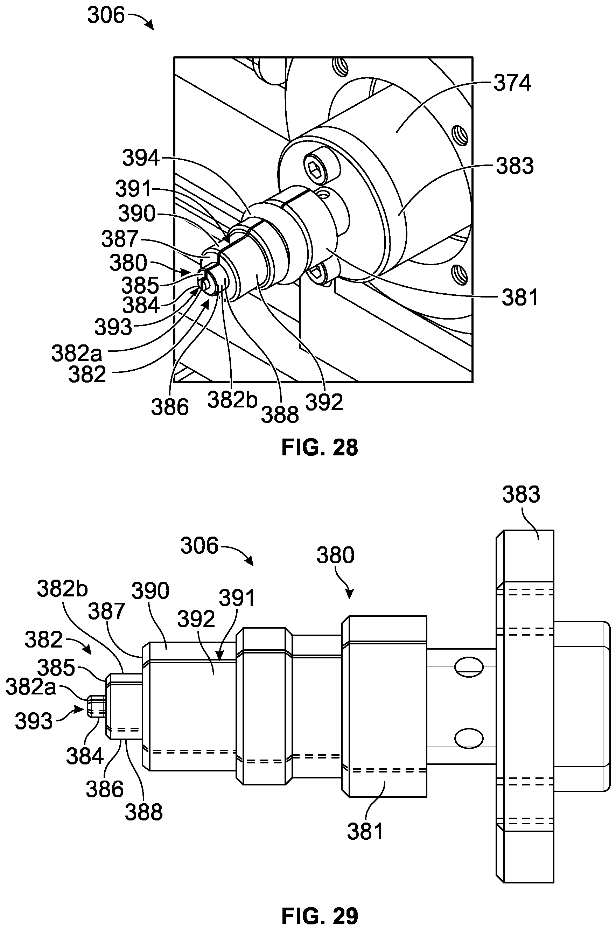

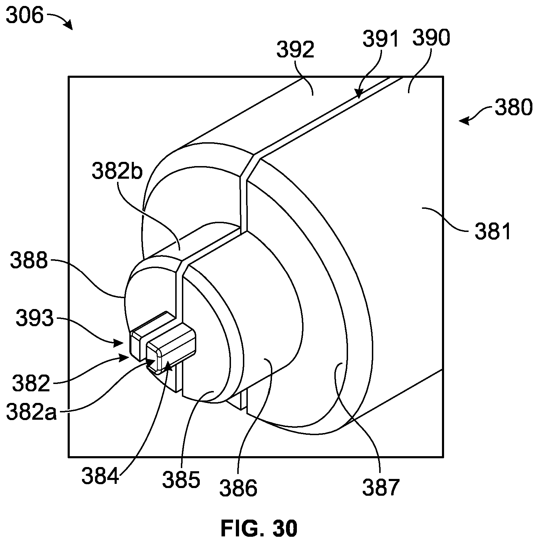

Figure 28 is a front perspective view of a portion of the ring holder 306 showing the ring mandrel 380 in accordance with an exemplary embodiment. Figure 29 is a side view of the ring mandrel 380 in accordance with an exemplary embodiment. Figure 30 is a front perspective view of a portion of the ring mandrel 380 in accordance with an exemplary embodiment. The ring mandrel 380 includes a body 381 having at least one ring mount 382 at the front of the body 381 used to support the pull ring 30. The ring mandrel 380 includes a mounting bracket 383 and a rear of the body 381 used to mount the ring mandrel 380 to the ring gantry 370, such as to the rotating mount 374. In the illustrated embodiment, the ring mandrel 380 includes multiple ring mounts 382a, 382b used to support different size pull rings 30. For example, the ring mounts 382a, 382b have different diameters to hold different diameter pull rings 30 without the need to change out the components of the ring holder 306 to accommodate the different sized pull rings 30.

In an exemplary embodiment, the ring mandrel 380 includes a first step 384 defining the first ring mount 382a and a second step 386 defining the second ring mount 382b. The first and second steps 384, 386 may be coincident with a central axis of the ring mandrel 380. The first and second steps 384, 386 have outer surfaces 388 extending axially (for example, parallel to the central axis of the ring mandrel 380). In an exemplary embodiment, the outer surfaces 388 are cylindrical, but have different diameters. The ring mandrel 380 includes a first end wall 385 and a second end wall 387. The first step 384 extends forward from the first end wall 385. The second step 386 extends forward from the second end wall 387, between the first end wall 385 and the second end wall 387. The first step 384 has a first diameter, and the second step 386 has a second diameter larger than the first diameter. The first and second steps 384, 386 have different diameters to receive different size pull rings 30. The ring mandrel 380 may include greater or fewer steps in alternative embodiments defining corresponding ring mounts 382 configured to hold different sized pull rings 30.

In an exemplary embodiment, the ring mandrel 380 is expandable. For example, the ring mandrel 380 may expand and contract to increase or decrease the size of the ring mount 382. For example, when the ring mandrel 380 is contracted, the pull ring 30 may easily fit over the ring mount 382. After the pull ring 30 is located around the ring mount 382, the ring mandrel 380 may be expanded to hold the pull ring 30 by a compression fit. For example, the ring mandrel 380 may press outward against the inner surface of the pull ring 30 to hold the pull ring 30 on the ring mount 382. In an exemplary embodiment, the ring mandrel 380 includes a first mandrel member 390 and a second mandrel member 392 meeting at a seam 391 located between the first and second mandrel members 390, 392. The first and second mandrel members 390, 392 are movable relative to each other to expand the size of the ring mount 382 to hold the pull ring 30 on the ring mount 382. The first and second mandrel members 390, 392 are movable relative to each other to reduce the size of the ring mount 382 to release the pull ring 30 from the ring mount 382. In the illustrated embodiment, the seam 391 extends from a front 393 of the ring mandrel 380 at least partially along the length of the ring mandrel 380. In an exemplary embodiment, the seam 391 does not extend the entire length of the ring mandrel 380. Rather, a portion of the body 381 is whole (seamless) supporting the first and second mandrel members 390, 392 and allowing the first and second mandrel members 390, 392 to spread apart to expand the size of the ring mount 382.

In an exemplary embodiment, an elastic ring 394 surrounds the ring mandrel 380 compressing the first and second mandrel members 390, 392 toward each other. The elastic ring 394 may be stretched when the first and second mandrel members 390, 392 are spread apart and allowed to expand against the inward compression of the elastic ring 394.

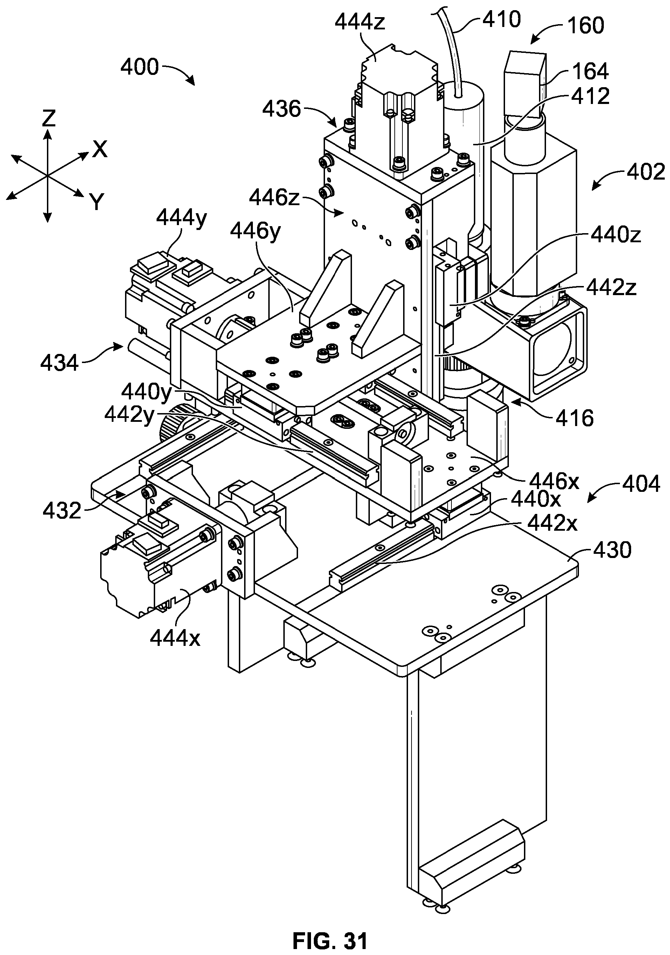

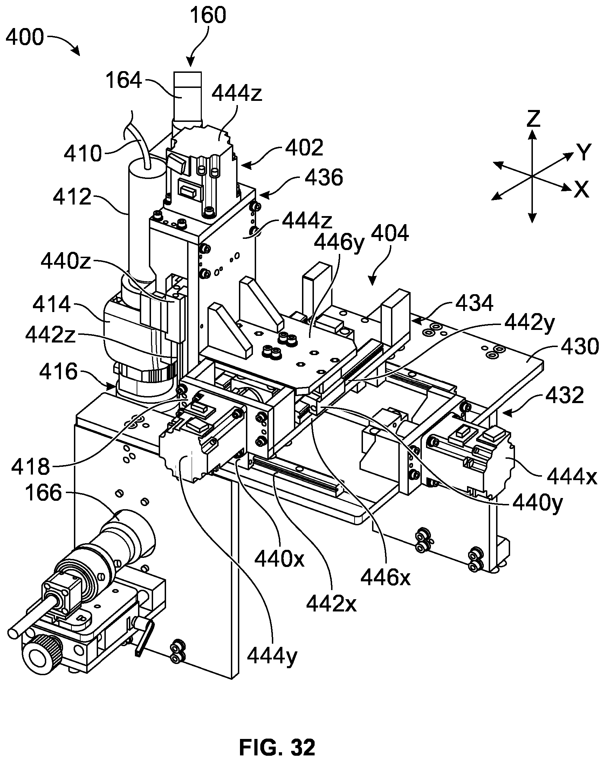

Figure 31 is a front perspective view of the laser weld system 400 in accordance with an exemplary embodiment. Figure 32 is a rear perspective view of the laser weld system 400 in accordance with an exemplary embodiment. The laser weld system 400 includes a laser head assembly 402 and a laser head gantry 404. The laser had assembly 402 is used to laser weld the pull wire 20 to the pull ring 30. The laser head gantry 404 is used to move the laser head assembly 402 during the laser welding process. The laser weld system 400 is located at the assembly zone 102 of the pull wire assembly machine 100. In an exemplary embodiment, components of the vision system 160 are incorporated into the laser weld system 400 to control operation of the laser weld system 400 during the laser welding process. For example, the laser head gantry 404 is controlled based on images from the vision system 160.

The laser head assembly 402 includes an optical fiber 410, a collimator 412, a turning mirror 414, and an argon nozzle 416. The laser head assembly 402 is used to create a laser beam for laser welding. In an exemplary embodiment, the laser had assembly 402 includes a ring light 418 to illuminate the assembly zone 102 for imaging by the vision system 160.

In an exemplary embodiment, the vision system 160 includes a top camera 164 and a side camera 166 for imaging the assembly zone 102 from different angles. Images from the cameras 164, 166 are used to control other components of the pull wire assembly machine 100. For example, the images may be used for positioning the laser had assembly 402. The images may be used for positioning the whole wire 20 using the wire placement system 200 and/or positioning the pull ring 30 using ring placement system 300.

In an exemplary embodiment, the laser head gantry 404 includes a gantry platform 430, an X-positioner 432, a Y-positioner 434, and a Z-positioner 436. The X-positioner 432 is movable in the X direction. The Y-positioner 434 is movable in the Y direction. The Z-positioner 436 is movable in the Z direction. In an exemplary embodiment, each of the-positioners 432, 434, 436 include a slide 440 (440x, 440y, 440z, respectively) slidably coupled to a rail 442 (442x, 442y, 442z, respectively) and an actuator 444 (444x, 444y, 444z, respectively) operably coupled to the slide 440 to move the slide 440 on the rail 442. The-positioners 432, 434, 436 may each include a platform 446 (446x, 446y, 446z, respectively) used to support other components. The rail 442x of the X-positioner 432 is oriented parallel to the X direction to allow movement of the slide 440x in the X direction. The rail 442y of the Y-positioner 434 is oriented parallel to the Y direction to allow movement of the slide 440y in the Y direction. The rail 442z of the Z-positioner 436 is oriented parallel to the Z direction to allow movement of the slide 440z in the Z direction.

In the illustrated embodiment, the X-positioner 432 is mounted to the gantry platform 430, the Y-positioner 434 is mounted to the platform 446x of the X-positioner 432, the Z-positioner 436 is mounted to the platform 446y of the Y-positioner 434, and the laser head assembly 402 is mounted to the platform 446z of the Z-positioner 436. Other arrangements are possible in alternative embodiments. The actuators 444 may be electrically actuated, hydraulically actuated, or pneumatically actuated. The actuators 444 are operably coupled to the control system 150 to control positioning of the components, such as for positioning the laser head assembly 402 relative to the pull ring 30 and the pull wire 20 for the laser welding process. The actuators 444 may be controlled based on signals or inputs from the vision system 160, such as images from the cameras 164, 166.

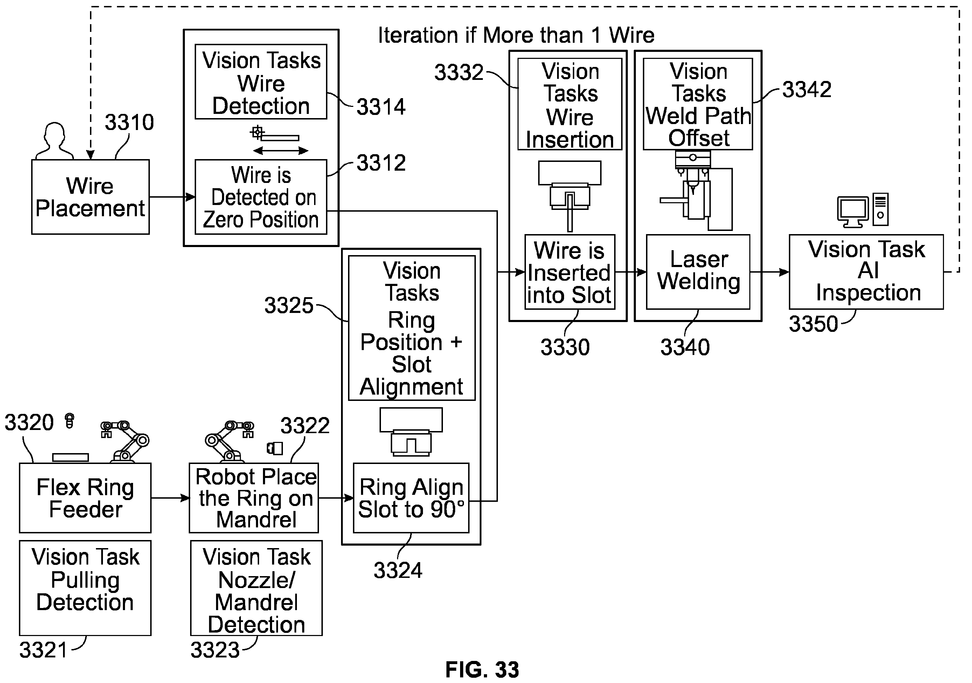

Figure 33 illustrates an assembly method for assembling the pull wire assembly 10 in accordance with an exemplary embodiment showing vision tasks performed by the vision system 160.

At step 3310, the method includes placing the pull wire 20 into the pull wire assembly machine 100. The pull wire 20 may be manually loaded into the wire placement system 200, such as into the wire guide module 202 by the operator, such as through the wire load port 134 at the front of the cabinet 110. At step 3312, the method includes positioning the pull wire 20 to the assembly zone 102. The wire placement system 200 may be used to position the pull wire 20 at the assembly zone, such as using the wire pull module 204 to advance the pull wire 20 to the wire insertion module 206. At 3314, the vision system 160 performs wire detection to detect the position of the pull wire 20 at the assembly zone 102. For example, the cameras 164, 166 may be used to image the end of the pull wire 20 to determine the position of the pull wire 20. The vision system 160 may use boundary recognition to detect boundaries of the pull wire 20 (for example, top, bottom, side, end).

At step 3320, the method includes picking the pull ring 30 from a ring feeder device, such as by the ring placement system 300. For example, the pull ring 30 may be picked from the ring feeder 302 using the ring manipulator 304. At step 3321, the vision system 160 performs pull ring detection to determine the position(s) of the pull ring(s) 30 on the ring feeder 302 to control operation of the ring manipulator 304 during the pick process. For example, the feeder camera 162 may image the pull rings 30 at the ring feeder 302. At step 3322, the method includes placing the pull ring 30 on the ring mandrel 380. The pull ring 30 may be placed by the robotic arm 320 of the ring manipulator 304. At step 3323, the vision system 160 performs nozzle and/or mandrel detection to determine the position(s) of the vacuum nozzle 342 and/or the ring mandrel 380 to control placing of the pull ring 30 on the end of the ring mandrel 380. For example, the cameras 164, 166 may image the positions of the components during the placing operation. At step 3324, the method includes orienting the pull ring 30 in the assembly zone. The pull ring 30 may be rotated to a predetermined position in the assembly zone. For example, the slot 40 may be aligned with the pull wire 20 at the home position. In various embodiments, the slot 40 of the pull ring 30 may be oriented in an upright or 90° position. At step 3325, the vision system 160 performs pull ring orientation detection to determine the orientation of the pull ring 30 at the assembly zone 102. For example, the cameras 164, 166 may image the pull ring 30 to determine the orientation of the slot 40 of the pull ring 30, such as to align the slot in a particular orientation, such as facing vertically upward at the 90° position. The vision system 160 may use boundary recognition to detect boundaries of the pull ring 30 (for example, top, bottom, side, end). The vision system 160 may detect the location/orientation of the slot 40. The vision system 160 may calculate an amount of rotation needed to move the pull ring 30 from the detected position to the final (for example, 90° position) based on the imaging and the slot detection. The vision system 160 may determine the diameter of the pull ring 30 based on the imaging. The vision system 160 may determine the position of the pull ring 30, such as for operating the ring gantry 370 to position the pull ring 30 within the assembly zone 102.

At step 3330, the method includes inserting the pull wire 20 into the slot 40 of the pull ring 30. The wire placement system 200 may be used to advance the pull wire 20 into the slot 40 of the pull ring 30. At step 3332, the vision system 160 performs wire insertion imaging to control insertion of the pull wire 20 into the slot 40 of the pull ring 30. The images are used to align the end of the pull wire 20 with the slot 40 during the insertion process. For example, the cameras 164, 166 may image the positions of the pull wire 20 and the pull ring 30 during the insertion operation. The wire insertion module 206 and/or the ring holder 306 may be controlled (for example, moved) based on the images to align and insert the pull wire 20 and the pull ring 30.

At step 3340, the method includes laser welding the pull wire 20 to the pull ring 30. The laser weld system 400 may be used to laser weld the pull wire 20 to the pull ring 30. At step 3342, the vision system 160 performs laser welding imaging to determine the position of the pull wire 20/pull ring 30 and/or to determine the position of the laser head assembly 402. The images are used to position the laser head assembly 402 during the laser welding process. For example, the cameras 164, 166 may image the positions of the components during the laser welding operation.

At step 3350, the method includes a quality inspection of the pull wire assembly 10. For example, the laser weld joint between the pull wire 20 and the pull ring 30 may be inspected by the vision system 160 to determine if the laser weld passes or fails the quality inspection. The cameras 164, 166 may be used for the quality assessment or another dedicated camera for the AI inspection may be provided.

Figure 34 is a front perspective view of the pull wire assembly removal system 500 in accordance with an exemplary embodiment. The pull wire assembly removal system 500 includes the transport device 502, the sorting tray assembly 504, and the storage tray assembly 506. The transport device 502 is configured to gather the assembled pull wire assembly 10 and transport the pull wire assembly 10 from the assembly zone 102 to the removal zone 104. The transport device 502 releases the pull wire assembly 10 into the sorting tray assembly 504. The sorting tray assembly 504 is used to sort the pull wire assembly 10 into the storage tray assembly 506. For example, the pull wire assemblies 10 may be sorted based on a quality assessment, such as based on a determination that the pull wire assembly 10 passes or fails a quality threshold. The pull wire assemblies 10 may be sorted based on a size (for example, diameter) of the pull ring 30 and/or length of the pull wire 20. The pull wire assemblies 10 are removed into the storage tray assembly 506. In an exemplary embodiment, multiple pull wire assemblies 10 may be stored in the storage tray assembly 506 prior to removal from the pull wire assembly machine 100. The removal system 500 may allow removal of the pull wire assemblies 10 by opening the collection ports 136, 138 (Figure 1) to access the storage tray assembly 506 and remove the pull wire assemblies 10 from the storage tray assembly 506.

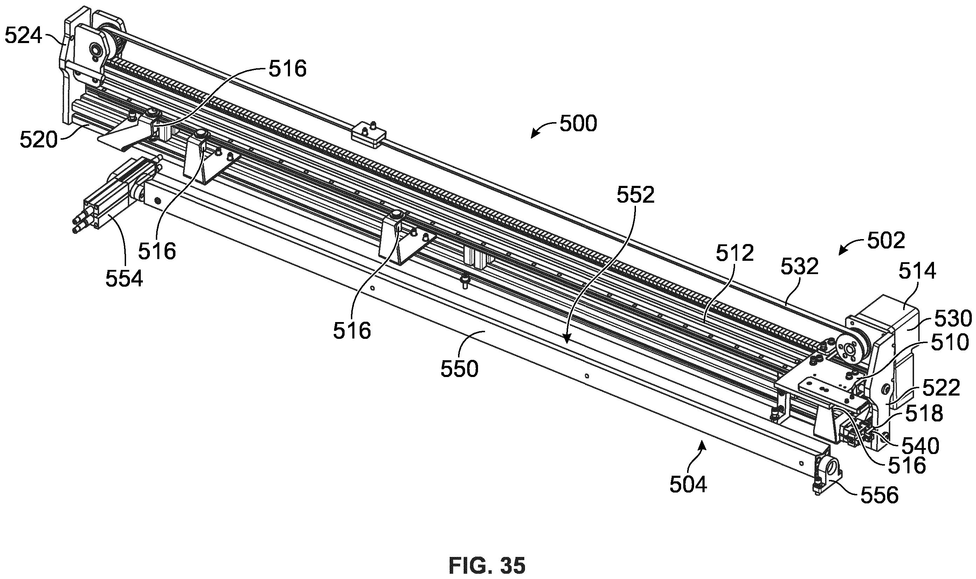

Figure 35 is a perspective view of a portion of the pull wire assembly removal system 500 showing the transport device 502 and the sorting tray assembly 504 located adjacent the transport device 502. The transport device 502 includes a cart 510 slidably coupled to a rail 512 and an actuator 514 operably coupled to the cart 510 to move the cart 510 on the rail 512. In an exemplary embodiment, the transport device 502 includes one or more position sensors 516 configured to sense the position of the cart 510 along the rail 512. The actuator 514 may be controlled based upon signals from the position sensors 516. In an exemplary embodiment, the transport device 502 includes an holding member 518 coupled to the cart 510 and movable with the cart 510. The holding member 518 is configured to pick up the pull wire assembly 10 and move the pull wire assembly 10 through the removal system 500. For example, the holding member 518 may move the pull wire assembly 10 from the assembly zone 102 to the removal zone 104.

The rail 512 may include a track to guide movement of the cart 510 on the rail 512. For example, the rail 512 includes one or more rail members 520 extending between a front plate 522 and the rear plate 524. The rail members 520 include tracks to guide movement of the cart 510 along the rail members 520. The cart 510 may be movable in a linear direction, such as in a direction parallel to the axis of the pull wire 20 (for example, front to rear).

In an exemplary embodiment, the actuator 514 is coupled to the front plate 522 and/or the rear plate 524. The actuator 514 includes a motor 530 and a pulley 532 operably coupled to the motor 530. The motor 530 is operated to move the pulley 532, such as in a forward direction or a rearward direction. The cart 510 is coupled to the pulley 532 and is movable by the pulley 532. The holding member 518 is moved with the cart 510 between a first position and a second position. The first position is a forward position, such as located proximate to the front plate 522. The second position is a rear position, such as located near the rear plate 524. The holding member 518 is configured to hold the assembled pull wire assembly 10 to move the pull wire assembly 10 from the first position to the second position. The first position corresponds to the assembly zone 102 and the second position corresponds to the removal zone 104. The holding member 518 is configured to pick up the pull wire assembly 10 from the assembly zone 102 and move the pull wire assembly 10 to the removal zone 104 where the pull wire assembly 10 is released to the sorting tray assembly 504. For example, the pull ring 30 is configured to be picked up by the holding member 518 and moved in a rearward direction to pull the pull wire 20 through the pull wire assembly machine 100 to move the assembled pull wire assembly 10 into the sorting tray assembly 504.

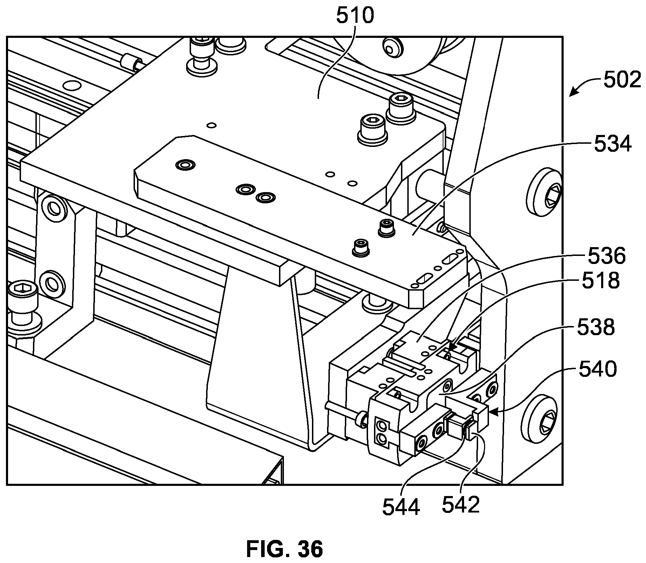

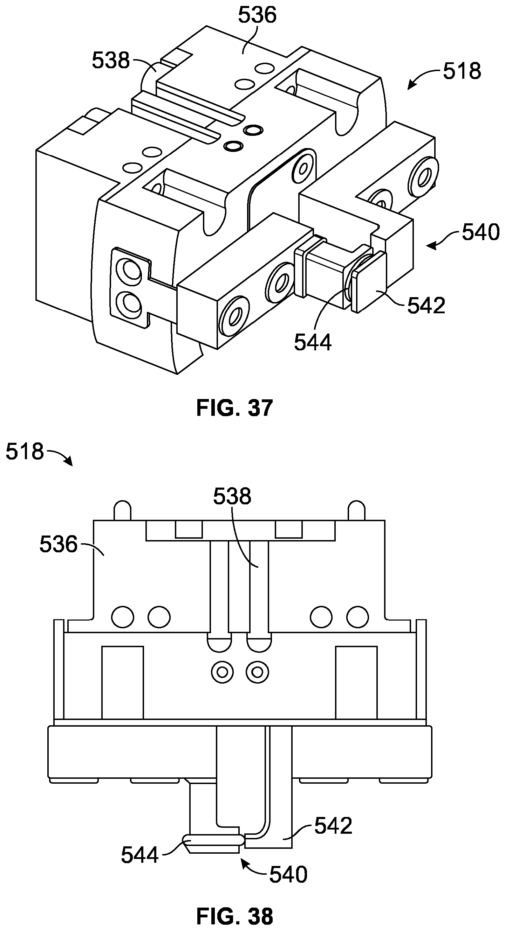

Figure 36 is an enlarged view of a portion of the transport device 502 showing the holding member 518 in accordance with an exemplary embodiment. Figure 37 is a perspective view of the holding member 518 in accordance with an exemplary embodiment. Figure 38 is a top view of the holding member 518 in accordance with an exemplary embodiment. In an exemplary embodiment, the cart 510 includes a support arm 534. The holding member 518 is coupled to the support arm 534.

The holding member 518 includes a mounting block 536 and a gripper 540 with one or more gripper elements 542, such as gripper fingers, configured to grip the pull wire assembly 10 and move the pull wire assembly 10 within the removal system 500. In an exemplary embodiment, the holding member 518 includes an actuator 538 operably coupled to the gripper 540 to close and open the gripper elements 542, such as to grip and release the pull ring 30. For example, the gripper elements 542 may be rotatably coupled to the mounting block 536 and are configured to rotate between the closed and opened positions. The actuator 538 may be electrically actuated, hydraulically actuated, or pneumatically actuated. The actuator 538 is operably coupled to the control system 150 to control opening and closing of the gripper 540. In an exemplary embodiment, the gripper 540 includes an O-ring 544 coupled to one or more of the gripper elements 542. The O-ring 544 is soft and pliable to prevent damage to the pull ring 30 when the gripper elements 542 close against the pull ring 30. The O-ring 544 may be manufactured from a rubber material to provide sliding friction to prevent dropping of the pull ring 30 from the gripper 540.

With reference back to Figure 35, the sorting tray assembly 504 is located at the removal zone 104. In the illustrated embodiment, the sorting tray assembly 504 is located directly below the transport device 502. The pull wire assembly 10 may be dropped into the storage tray assembly 504 when the holding member 518 releases the pull wire assembly 10. The sorting tray assembly 504 includes a sorting tray 550 having a sorting channel 552. The sorting tray assembly 504 includes a sorting tray actuator 554 operably coupled to the sorting tray 550 to move the sorting tray 550 between various positions. In an exemplary embodiment, the sorting tray assembly 504 includes pivot mounts 556 at the front and rear ends of the sorting tray 550. The sorting tray 550 is pivotably coupled to the pivot mounts 556. The storage tray actuator 554 is configured to pivot the storage tray 550 on the pivot mounts 556 between the various positions.

In an exemplary embodiment, the sorting tray 550 is movable between a home position (shown in Figure 35), a first removal position, and a second removal position. The sorting tray 550 may be rotated in a first direction (for example, clockwise) from the home position to the first removal position and may be rotated in a second direction (for example, counterclockwise) from the home position to the second removal position. In the illustrated embodiment, the sorting channel 552 is located at the top of the sorting tray 550 in the home position. As the sorting tray 550 is moved to the first removal position, the pull wire assembly 10 may be moved in a first direction (for example, to the left). As the sorting tray 550 is moved to the second removal position, the pull wire assembly 10 may be moved in a second direction (for example, to the right). In an exemplary embodiment, the pull wire assemblies 10 are sorted by the sorting tray assembly 504 based on a quality assessment, such as based on a determination that the pull wire assembly 10 passes or fails a quality threshold. The vision system 160 may be used for the quality assessment. For example, images of the laser weld between the pull wire 20 and the pull ring may be analyzed, such as by an AI inspection module, to determine if the laser weld is of good quality (uniform weld joint and/or continuous for a sufficient weld length) or bad quality (missing weld joint and/or improperly located wire and the like). The pull wire assemblies 10 may be sorted based on size of the pull ring 30 and/or length of the pull wire 20.

In an exemplary embodiment, the transport device 502 is used to load the assembled pull wire assembly 10 into the sorting tray 550 at the removal zone 104. For example, the cart 510 and the holding member 518 are moved in the forward direction to a pickup position (shown in Figure 35) to pick up the pull wire assembly 10. In an exemplary embodiment, one of the position sensors 516 is located at the front of the transport device 502 to define the pickup position. The actuator 514 is operated to move the cart 510 in the forward direction until the cart 510 is sensed by the position sensor 516. The actuator 514 stops when the cart 510 is at the pickup position. The gripper 540 picks up the pull wire assembly 10 from the assembly zone 102 at the pickup position. The actuator 514 then moves the cart 510 rearward to an end position. One or more position sensors 516 may be located proximate to the rear of the transport device 502 to define the end position. Optionally, multiple position sensors 516 may be provided to define different end positions, such as corresponding to different length pull wires 20. The cart 510 stops at the appropriate position sensor 516. The gripper 540 releases the pull wire assembly 10 into the sorting tray 550 at the removal zone 104. After the pull wire assembly 10 is released, the cart 510 may return to a home position. The home position may be located between the pickup position and the end position. One of the position sensors 516 may be located at an appropriate location between the front and the rear to define the home position. The home position is located remote from the pickup position such that the cart 510 and the holding member 518 are located out of the assembly zone 102 so as to not interfere with the assembly process of the pull wire assembly 10.

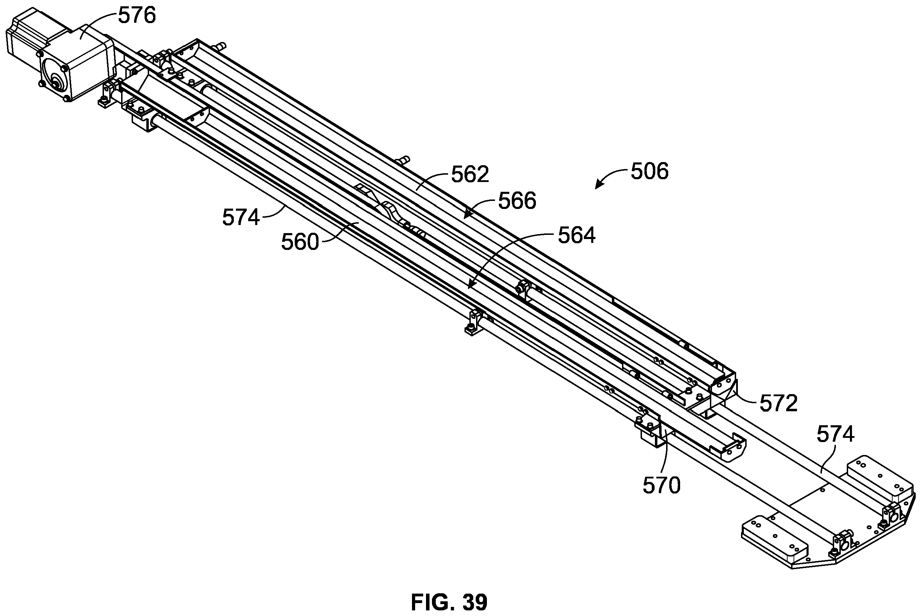

Figure 39 is a perspective view of the storage tray assembly 506 in accordance with an exemplary embodiment. The storage tray assembly 506 is used to store the pull wire assemblies 10 after the pull wire assemblies 10 are sorted by the sorting tray assembly 504. In an exemplary embodiment, the storage tray assembly 506 includes a first storage tray 560 and a second storage tray 562 at the removal zone 104. The first storage tray 560 includes a first channel 564 used to store the pull wire assemblies 10. The second storage tray 562 includes a second channel 566 used to store the pull wire assemblies 10. In an exemplary embodiment, the pull wire assemblies 10 are sorted by the sorting tray assembly 504 based on a quality assessment, such as based on a determination that the pull wire assembly 10 passes or fails a quality threshold. The pull wire assemblies 10 may be sorted based on size of the pull ring 30 and/or length of the pull wire 20. The pull wire assemblies 10 are deposited into the first and second storage trays 560, 562 based on the sorting criteria. For example, the first storage tray 560 may receive the pull wire assemblies 10 that pass the quality assessment when the sorting tray 550 is moved to the first removal position to deposit the pull wire assemblies 10 in the first direction, whereas the second storage tray 562 may receive the pull wire assemblies 10 that fail the quality assessment when the sorting tray 550 is moved to the second removal position to deposit the pull wire assemblies 10 in the second direction,. As such, the first storage tray 560 is a PASS storage tray and the second storage tray 562 is a FAIL storage tray. In an exemplary embodiment, the first and second storage trays 560, 562 are offset from the sorting tray 550, such as shifted to opposite sides of the sorting tray 550 to receive the corresponding pull wire assemblies 10 when the sorting tray 550 is either rotated in the first direction or the second direction. The pull wire assemblies 10 may be stored in the storage trays 560, 562, such as using magnets arranged in the storage trays 560, 562.

In an exemplary embodiment, the storage tray assembly 506 includes a first slide 570 supporting the first storage tray 560 and a second slide 572 supporting the second storage tray 562. The first and second slides 570, 572 are slidable along rails 574 to allow the storage trays 560, 562 two pull out from the removal system 500, such as for the operator to gather the pull wire assemblies 10 held in the storage trays 560, 562. For example, the collection ports 136, 138 (shown Figure 1) may be opened to access the storage trays 560, 562. In an exemplary embodiment, an actuator 576 is used to move the storage trays 560, 562 from a storage position to an access position. The actuator 576 may be operated, such as based on a call operation by the operator, such as at the user interface. The collection ports 136, 138 may be opened by the operator to access the storage trays 560, 562. The actuator 576 may be used to return the storage trays 560, 562 to the storage position.

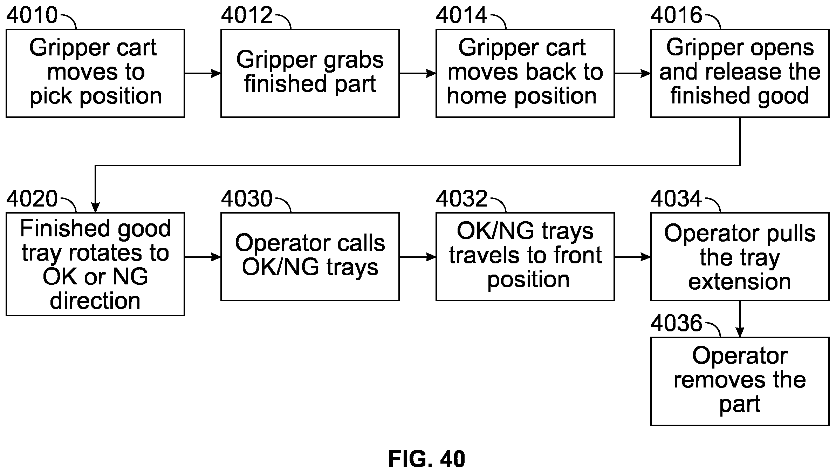

Figure 40 illustrates a removal method for removing the pull wire assemblies 10 from the pull wire assembly machine 100. At step 4010, the method includes moving the cart 510 and the gripper 542 the pickup position. The position sensor 516 at the pickup position may be used to control the positioning of the cart 510 and the gripper 540. At step 4012, the method includes grabbing the pull wire assembly 10 with the gripper 540. At step 4014, the method includes moving the cart 510 and the gripper 540 in a rearward direction to the end position. The position sensor(s) 516 at the end positions may be used to control the positioning of the cart 510 in the gripper 540. At step 4016, the method includes opening the gripper 542 release the pull wire assembly 10 to the sorting tray 550.

In an exemplary embodiment, the pull wire assembly 10 is imaged to determine the quality of the laser weld. In various embodiments, the laser weld is imaged prior to the pull wire assembly 10 being picked up by the gripper 540. At step 4020, the method includes rotating the sorting tray 550 to sort the pull wire assembly 10 in the storage tray assembly 506. For example, the sorting tray 550 may be rotated in a first direction if the pull wire assembly 10 is of good quality and may be rotated in a second direction if the pull wire assembly 10 is of poor quality to deposit the into the different storage trays 560, 562 of the storage tray assembly 506.

At step 4030, the operator retrieves the pull wire assemblies 10 from the first storage tray 560 and/or the second storage tray 562. For example, the operator may open the collection port 136 and/or the collection port 138 to access the corresponding storage trays 560, 562. At step 4032, the actuator 576 is operated to move the storage tray 560 and/or the storage tray 562 two a forward position. At step 4034, the operator pulls the slide 570 and/or the slide 572 to extend the storage tray 560 and/or the storage tray 562 to expose the pull wire assemblies 10 in the storage trays 560, 562. At step 4036, the operator removes the pull wire assemblies 10 from the storage trays 560, 562. The operator may then push the storage trays 560, 562 back into the cabinet 110 to close the access doors at the collection ports 136, 138.

The pull wire assembly machine 100 automates the assembly process of the pull wire assemblies 10. For example, the pull wire assembly machine 100 automatically positions the pull wire 20 and the pull ring 30 for the laser welding process. The pull wire assembly machine 100 automates the laser welding process. The pull wire assembly machine 100 uses imaging to assess the quality of the laser weld between the pull wire 20 and the pull ring 30. In an exemplary embodiment, the pull wire assembly machine 100 supports the pull wire assemblies 10 based on the quality assessment for the operator to gather the pull wire assemblies 10 for further processing. The assembly process for the pull wire assemblies 10 is repeatable and may be performed at high quality with limited waste by automating the assembly process. The assembly process may be completed quickly to increase throughput of the product. For example, the automated assembly process may be completed in under 20 seconds for a single wire assembly or under 40 seconds for a dual wire assembly compared to the manual assembly process, which typically takes approximately 75 seconds for a single wire assembly or 150 seconds for a dual wire assembly.

Therefore, there is provided a ring holder for a pull wire assembly machine as defined in any of the following numbered clauses:
Clause 13. A ring holder for a pull wire assembly machine configured to assemble a pull wire to a pull ring, the ring holder comprising:
   a ring mandrel having a ring mount at a front of the ring mandrel configured to hold the pull ring; and
   a ring gantry supporting the ring mandrel, the ring gantry movable in three dimensional space to position the ring mandrel to align the pull ring with the pull wire for assembly of the pull wire and the pull ring.
Clause 14. The ring holder of Clause 13, wherein the ring gantry includes a base, an X slide configured to move the ring mandrel in an X direction relative to the base, a Y slide configured to move the ring mandrel in a Y direction relative to the base, and a Z slide configured to move the ring mandrel in a Z direction relative to the base, wherein the X, Y, and Z directions are mutually perpendicular directions.
Clause 15. The ring holder of Clause 13 or 14, wherein the ring gantry includes a rotating mount configured to rotate relative to the base, the ring mandrel being mounted to the rotating mount, the rotating mount configured to rotate the ring mandrel to rotate the pull ring relative to the pull wire.
Clause 16. The ring holder of Clause 14 or 15, wherein the X slide is coupled to the base, the Y slide is coupled to the X slide, and the Z slide is coupled to the Y slide.
Clause 17. The ring holder of any one of Clauses 13 to 16, wherein the first and second mandrel members are movable relative to each other to expand a size of the ring mount to hold the pull ring on the ring mount, and wherein the first and second mandrel members being movable relative to each other to reduce a size of the ring mount to release the pull ring from the ring mount.

There is also provided a pull wire assembly machine configured to assemble a pull wire assembly from a pull wire and a pull ring as defined in any of the following numbered clauses:
Clause 18. A pull wire assembly machine configured to assemble a pull wire assembly from a pull wire and a pull ring, the pull wire assembly machine comprising:
   a ring placement system having a ring holder configured to position the pull ring at an assembly zone, the ring holder including a ring mandrel having a first mandrel member and a second mandrel member meeting at a seam, the ring mandrel including a ring mount at a front of the ring mandrel configured to hold the pull ring, wherein the first and second mandrel members are movable relative to each other to expand a size of the ring mount to hold the pull ring on the ring mount and the first and second mandrel members being movable relative to each other to reduce a size of the ring mount to release the pull ring from the ring mount, the ring holder including a ring gantry supporting the ring mandrel, the ring gantry movable in three dimensional space to position the ring mandrel within the assembly zone;
   a wire placement system configured to position the pull wire at the assembly zone; and
   a laser weld system at the assembly zone configured to laser weld the pull wire to the pull ring to form a pull wire assembly.
Clause 19. The pull wire assembly machine of Clause 18, wherein the ring placement system includes a ring manipulator configured to load the pull ring onto the ring mandrel at the assembly zone, the ring manipulator including a robotic arm movable in three-dimensional space and an end effector at a distal end of the robotic arm, the end effector including a vacuum nozzle including a vacuum chamber configured to receive the pull ring and hold the pull ring in the vacuum chamber.
Clause 20. The pull wire assembly machine of Clause 18 or 19, wherein the ring placement system includes a ring feeder configured to support a plurality of the pull rings and a ring manipulator configured to pick the individual pull rings from the ring feeder and place the picked pull ring on the ring mandrel at the assembly zone.
Clause 21. The pull wire assembly machine of Clause 18, 19 or 20, further comprising a pull wire assembly removal system configured to allow removal of the pull wire assembly from the pull wire assembly machine.

## Claims

1. A ring holder (306) for a pull wire assembly machine (100) configured to assemble a pull wire (20) to a pull ring (30), the ring holder comprising:
a ring mandrel (380) having a first mandrel member (390) and a second mandrel member (392) meeting at a seam (391), the ring mandrel including a ring mount (382) at a front (393) of the ring mandrel configured to hold the pull ring, wherein the first and second mandrel members are movable relative to each other to expand a size of the ring mount to hold the pull ring on the ring mount and the first and second mandrel members being movable relative to each other to reduce a size of the ring mount to release the pull ring from the ring mount.

2. The ring holder (306) of claim 1, wherein the seam (391) extends along the ring mount (382).

3. The ring holder (306) of claim 1 or 2, wherein the ring mount (382) is generally cylindrical having a diameter matching an inner diameter of the pull ring (30).

4. The ring holder (306) of claim 1, 2 or 3, wherein the ring mount (382) includes an outer surface configured to receive and hold the pull ring (30).

5. The ring holder (306) of any preceding claim, wherein the ring mandrel (380) includes an expansion device between the first and second mandrel members (390, 392) to spread the first and second mandrel members apart and expand the ring mandrel at the seam (391).

6. The ring holder (306) of any preceding claim, wherein the ring mount (382) is a first ring mount (382a) having a first diameter to receive first pull rings (30) having the first diameter, the ring mandrel (380) including a second ring mount (382b) having a second diameter to receive second pull rings (30) having the second diameter.

7. The ring holder (306) of claim 6, wherein the ring mandrel (380) includes a first step (384) defining the first ring mount (382a) and a second step (386) rearward of the first step defining the second ring mount (382b).

8. The ring holder (306) of claim 6 or 7, wherein the ring mandrel (380) includes a first end wall (385) and a second end wall (387), the first ring mount (382a) extending forward from the first end wall, the second ring mount (382b) extending from the second end wall to the first end wall.

9. The ring holder (306) of any preceding claim, further comprising a ring gantry (370) supporting the ring mandrel (380), the ring gantry movable in three dimensional space to position the ring mandrel to align the pull ring (30) with the pull wire (20) for assembly of the pull wire in the pull ring.

10. The ring holder (306) of claim 9, wherein the ring gantry (370) includes a base (368), an X slide configured to move the ring mandrel (380) in an X direction relative to the base, a Y slide configured to move the ring mandrel in a Y direction relative to the base, and a Z slide configured to move the ring mandrel in a Z direction relative to the base, wherein the X, Y, and Z directions are mutually perpendicular directions.

11. The ring holder (306) of claim 10, wherein the ring gantry (370) includes a rotating mount configured to rotate relative to the base, the ring mandrel being mounted to the rotating mount, the rotating mount configured to rotate the ring mandrel to rotate the pull ring relative to the pull wire.

12. The ring holder of claim 10 or 11, wherein the X slide is coupled to the base, the Y slide is coupled to the X slide, and the Z slide is coupled to the Y slide.
